(19) Europäisches Patentamt
European Patent Office
Office européen des brevets

(11) **EP 1 442 298 B1**

(12) **FASCICULE DE BREVET EUROPEEN**

(45) Date de publication et mention
de la délivrance du brevet:
**20.02.2008 Bulletin 2008/08**

(21) Numéro de dépôt: **02767540.4**

(22) Date de dépôt: **01.07.2002**

(51) Int Cl.:
*G01N 33/542* (2006.01)    *C12Q 1/68* (2006.01)

(86) Numéro de dépôt international:
**PCT/FR2002/002283**

(87) Numéro de publication internationale:
**WO 2003/005028 (16.01.2003 Gazette 2003/03)**

(54) **PROCEDE DE SELECTION D'AGENTS BIO-ACTIFS PAR COUPLAGE DE LUMINESCENCE ET SYSTEME CELLULAIRE VIVANT POUR LA MISE EN OEUVRE DE CE PROCEDE**

VERFAHREN ZUR AUSWAHL BIOAKTIVER WIRKSTOFFE DURCH LUMINESZENZKOPPLUNG UND LEBENDES ZELLENSYSTEM DAFÜR

METHOD FOR SELECTING BIOACTIVE AGENTS BY LUMINESCENCE COUPLING AND LIVING CELL SYSTEM THEREFOR

(84) Etats contractants désignés:
**AT BE BG CH CY CZ DE DK EE ES FI FR GB GR IE IT LI LU MC NL PT SE SK TR**

(30) Priorité: **02.07.2001 FR 0108758**

(43) Date de publication de la demande:
**04.08.2004 Bulletin 2004/32**

(73) Titulaire: **Novaleads**
**31520 Ramonville Saint Agne (FR)**

(72) Inventeurs:
• **TOCANNE, Jean-François**
**F-31130 Flourens (FR)**
• **FURGER, Christophe**
**F-31000 Toulouse (FR)**

(74) Mandataire: **Cabinet BARRE LAFORGUE & associés**
**95, rue des Amidonniers**
**31000 Toulouse (FR)**

(56) Documents cités:
EP-A- 0 977 035          WO-A-00/43780
WO-A-01/46691           WO-A-99/66324

• **MARJATTA RYTÖMAA ET AL: "dissociation of cytochrome c from liposomes by histone h1. Comparison with basic peptides" BIOCHEMISTRY, vol. 35, 1996, pages 4529-4539, XP002200468**
• **USTER P S ET AL: "RESONANCE ENERGY TRANSFER MICROSCOPY: OBSERVATIONS OF MEMBRANE-BOUND FLUORESCENT PROBES IN MODEL MEMBRANES AND IN LIVING CELLS" JOURNAL OF CELL BIOLOGY, ROCKEFELLER UNIVERSITY PRESS, NEW YORK, US, US, vol. 103, no. 4, octobre 1986 (1986-10), pages 1221-1234, XP000998288 ISSN: 0021-9525**

## Description

L'invention concerne un procédé de sélection d'agents bio-actifs -notamment mais non exclusivement d'agents pharmacologiquement actifs pour l'homme ou l'animal-. L'invention s'étend à un système cellulaire vivant, à l'exception d'un embryon humain, d'un corps humain et d'un élément non isolé d'un corps humain, adapté à la mise en oeuvre d'un tel procédé.

Il existe actuellement des banques d'agents potentiellement bio-actifs (molécules générées par chimie combinatoire, substances d'origine naturelle...) privées ou publiques comprenant un très grand nombre d'agents dont l'activité biologique vis-à-vis de cibles prédéterminées doit pouvoir être testée pour la sélection (criblage) d'agents biologiquement actifs, notamment susceptibles de fournir de nouveaux médicaments.

Dans ce but, il est nécessaire de disposer de moyens de criblage à haut débit. En particulier, dans les phases de développement des médicaments pour l'homme ou l'animal, chaque gain de temps représente un bénéfice substantiel.

Or, pour la sélection des agents bio-actifs, on utilise en pratique deux étapes successives de criblage : une étape de criblage primaire effectuée à haut débit (jusqu'à 100 000 molécules par jour) de façon automatisée, basée sur l'affinité des agents potentiellement actifs avec la cible moléculaire identifiée ou supposée être à l'origine de l'effet biochimique recherché, et permettant donc la sélection d'agents biochimiquement actifs sur la cible ; et une étape de criblage secondaire (ou "mécanistique") visant à apporter le plus d'informations possibles sur l'activité biologique réelle des agents potentiellement actifs (effets cellulaires ou toxiques et mécanismes intracellulaires moléculaires sous-jacents), réalisée avec un faible débit (100 à 1000 molécules par jour) sur un modèle cellulaire exprimant la cible moléculaire, et permettant la sélection des agents bio-actifs parmi les agents biochimiquement actifs.

La qualité des informations obtenues lors de cette étape de criblage secondaire influence directement les chances de succès des phases subséquentes d'essais précliniques et cliniques, extrêmement longues (5 à 7 ans) et coûteuses. L'amélioration de cette étape de criblage secondaire, tant du point de vue qualitatif que de sa durée, représente donc un enjeu majeur dans l'industrie pharmaceutique et vétérinaire, et plus généralement dans la recherche et le développement d'agents biologiquement actifs.

L'invention vise, de façon générale, à résoudre ce problème.

WO 9507463 décrit une méthode permettant l'expression intracellulaire d'une protéine recombinante formée d'une protéine intracellulaire fusionnée à une GFP. Les GFP constituent une famille de protéines naturellement fluorescentes dont on sait qu'elles peuvent être couplées par fusion de gènes à d'autres polypeptides tels que des protéines solubles ou membranaires, sans affecter leur taux d'expression ni la formation du fluorophore.

Dès lors, pour pallier les inconvénients des tests antérieurs de criblage secondaire (utilisation de radioéléments ; purification des ligands et/ou protéines cibles ; défauts de fiabilité ; faible contenu informatif des résultats...), il a été récemment proposé d'utiliser une protéine fluorescente, la GFP, ou l'un de ses variants ou fragments, fluorescents, dans le cadre du criblage secondaire :

- soit pour la détection de l'activité induite de certaines protéines spécifiques telles que la protéine kinase A (par exemple WO 9623898) en cultivant une cellule exprimant la protéine fusionnée avec la GFP, et par mesure des changements d'intensité de fluorescence,
- soit pour la mesure quantitative de la redistribution d'une protéine de transduction intracellulaire fusionnée avec la GFP et par traitement et analyse d'image (WO 9845704),
- soit pour la détection des interactions entre un récepteur membranaire marqué avec une GFP et son ligand marqué avec un groupement fluorescent photocomplémentaire par mesures quantitatives du transfert d'énergie de fluorescence par résonance (FRET) (WO 9855873),
- soit pour la détection d'interactions entre deux peptides ou protéines en les marquant par des composants fluorescents et en mesurant le FRET (WO 9912033) ou la diminution de la durée de vie de l'état excité du fluorophore donneur (WO 0043780).

Les tests tels que celui proposé par WO 9623898 ne sont pas réellement utilisables en pratique. En effet, les modifications d'intensité de fluorescence peuvent être très faibles, donc très difficiles à détecter et surtout impossibles à analyser. En effet, de part son principe même, fondé uniquement sur des observations des changements d'intensité globale de fluorescence, le test proposé ne fournit aucune information quant aux mécanismes intracellulaires moléculaires à l'origine de la variation de l'intensité de fluorescence.

Le test proposé par WO 9845704 présente aussi l'inconvénient de ne pouvoir être mis en oeuvre qu'à condition d'avoir identifié une protéine de transduction spécifique dont la redistribution dans la cellule est suffisamment importante pour pouvoir être mesurée par comparaison d'images de fluorescence. Il s'agit donc d'une méthode qui est limitée dans ses applications et nécessite une comparaison d'images soit subjective (par l'homme), soit complexe et coûteuse et de fiabilité imparfaite (analyse par traitement d'images). En particulier, avec une analyse d'images, il existe toujours une plage d'incertitude importante dans la discrimination qualitative d'une activation ou d'une absence d'activation. Ainsi, un tel procédé ne permet pas de fournir ou de comparer les concentrations minimales d'activation. Dès lors, l'analyse quantitative supposée être obtenue dans ce document n'est possible qu'avec les agents bio-actifs qui induisent une réponse cellulaire forte quantitativement (redistribution notable d'une protéine principalement entre cytoplasme ou noyau), mais ne permet pas de sélectionner des agents induisant des réponses cellulaires plus fines. Or, en pratique, compte tenu notamment de la complexité des mécanismes de transduction intracellulaires, il est rare que l'effet d'un agent bio-actif se traduise nécessairement par une redistribution d'une protéine spécifique suffisamment importante pour être détectée par comparaison d'images.

WO 9855873 est quant à lui limité à la détection de l'interaction non covalente du ligand (agent à tester) avec le récepteur membranaire, mais ne permet pas d'expliciter la réponse cellulaire induite en terme de mécanisme intracellulaire moléculaire sous-jacent. Ainsi, ce test n'est pas applicable au criblage secondaire où la réponse cellulaire réelle doit être prise en compte pour minimiser les risques d'échecs lors des phases de développement subséquentes et obtenir des informations quantitatives sur l'efficacité des agents bio-actifs sur les cellules (seuils de concentrations, niveaux d'activation relatifs, réversibilité, spécificité de l'effet,...).

En outre, les tests basés sur la détection d'interactions protéine-protéine tels que celui décrit par WO 9912033 supposent une connaissance préalable précise et fiable des mécanismes intracellulaires de la réponse aux agents bio-actifs pour corréler l'interaction à une activation ou une inhibition. Or, cette connaissance fait le plus souvent défaut et constitue justement le but du criblage secondaire. En outre, ces tests sont lourds et complexes à mettre en oeuvre car ils nécessitent une construction de plusieurs protéines de fusion, la co-transfection de ces constructions, la connaissance des deux protéines et de leurs interactions spécifiques. Ils sont aussi trop spécifiquement limités à des couples particuliers de protéines intracellulaires qui interagissent entre elles.

WO 0043780 décrit une méthode permettant de détecter des changements d'état d'un constituant de la cellule en réponse à un signal cellulaire et ceci par détection du FRET entre le constituant et un anticorps (désigné par "sensor" dans le document) spécifique d'un des états dudit constituant; l'anticorps et le constituant étant tous deux marqués par des groupements photocomplémentaires.

Ce test, particulièrement complexe à mettre en oeuvre, suppose l'obtention d'anticorps de forte affinité et hautement spécifiques, capables de distinguer une modification structurelle particulière d'un constituant précis intervenant spécifiquement dans une réponse cellulaire recherchée. Plus adapté à l'analyse de lysats cellulaires, ce type de test est difficile à appliquer sur des cellules vivantes. En effet, dans ce cas, les anticorps marqués d'un premier groupement photocomplémentaire doivent être introduits dans les cellules vivantes ou bien être synthétisés par celles-ci. Non naturellement présents dans ces cellules, ces anticorps introduits par des méthodes plus ou moins invasives, peuvent perturber considérablement leur fonctionnement et leur structure, et peuvent ainsi diminuer la fiabilité des analyses.

Il est à noter que WO 0043780 prescrit pour ce genre de test une détection de la diminution de la durée de vie de l'état excité du fluorophore donneur (due au phénomène de FRET) pour détecter le phénomène de FRET, méthode censée être plus fiable que la seule observation d'une variation de fluorescence. L'analyse est toutefois lourde et nécessite un matériel hautement adapté. La durée des tests, quant à elle, peut être conséquente et inapte à un criblage à haut débit.

Dans ce contexte, l'invention vise à permettre de réaliser une sélection d'agents bio-actifs à haut débit applicable sur des cellules vivantes, de façon simple et économique, et apte à fournir des résultats de grande fiabilité, tout en étant applicable dans de nombreuses applications distinctes, notamment pour le criblage secondaire.

L'invention vise plus particulièrement à permettre l'obtention d'informations tant qualitatives (notamment sur les mécanismes intracellulaires moléculaires sous-jacents) que quantitatives (notamment le suivi dans le temps) précises et fiables pour la sélection des agents bio-actifs, y compris lorsque la réponse cellulaire se traduit par des modifications quantitativement faibles des concentrations ou des distributions de concentrations dans le cytoplasme.

Dans tout le texte, on adopte la terminologie suivante :

- "agent" : tout composé ou groupe de composés reliés par tout type de liaison (covalente, ionique, complexation, interactions faibles...) de façon à être solidaires dans leur distribution spatiale,
- "couplage de luminescence" : tout processus physique entre deux groupements photocomplémentaires, dépendant de leur distance relative, dans lequel une énergie lumineuse émise par l'un et/ou l'autre des deux groupements photocomplémentaires est modifiée selon que ces derniers sont proches l'un de l'autre d'une distance inférieure à une valeur critique (généralement comprise entre 1 et 10 nm), un couplage énergétique intervenant alors entre eux, ou lorsque ces groupements sont éloignés l'un de l'autre d'une distance supérieure à cette valeur critique ; il peut s'agir de transfert d'énergie de fluorescence par résonance ou FRET (LAKOVICZ JR, (1999), in Principles of Fluorescence Spectroscopy, Eds Kluwer Academic/Plenum Publishers, New-York, pp 368-391), de transfert d'énergie de bioluminescence par résonance ou BRET (Angers S, Salahpour A, Joly E, Hilairet S, Chelsky D, Dennis M and Bouvier M, (2000), Proceeding of the National Academy of Science USA, 97, 7, 3684-3689, "Detection of $\beta$2-adrenergic receptor dimerization in living cells using Bioluminescence Resonnance Energy Transfer"),
- "agent associé à une membrane cellulaire" : tout agent comprenant au moins un ligand membranairé lipophile inséré dans au moins une couche lipidique d'une membrane cellulaire, ou comprenant au moins l'un des lipides d'origine naturelle d'une membrane cellulaire,

- "spécifiquement recruté à la membrane cellulaire" : tout processus biologique et/ou chimique par lequel au moins une certaine quantité d'un agent est liée (après avoir été produite) au moins à certains agents associés à la membrane cellulaire ; au contraire, ledit agent n'est pas spécifiquement recruté à la membrane cellulaire, soit lorsque ladite quantité de l'agent n'est pas produite, soit lorsqu'elle ne se lie pas à des agents associés à la membrane cellulaire,
- "marquage" et ses dérivés ("marquer", "marqué"...) : le fait de grouper en un agent unique, dit agent marqué, deux agents dont un au moins est adapté pour pouvoir être repéré dans l'espace ; le marquage peut être réalisé (par exemple par synthèse) directement, ou, au contraire, être réalisé indirectement par exemple par voie génétique en faisant exprimer l'agent marqué par des cellules transfectées avec une séquence nucléotidique appropriée,
- "signal cellulaire" : toute modification (du métabolisme, de la croissance, d'une activité intracellulaire ou extracellulaire...) d'un système cellulaire vivant induite par un agent ou phénomène,
- "système cellulaire vivant" : tout système ou milieu naturel ou artificiel contenant au moins une cellule vivante,
- "luminophore": tout groupement jouant un rôle de donneur et/ou d'accepteur, émetteur et/ou absorbant, dans un couplage de luminescence,
- "préserver au moins sensiblement les propriétés d'un système cellulaire vivant" : maintenir les fonctions vitales d'au moins une cellule vivante du système cellulaire vivant de façon suffisante pour permettre une réaction de la cellule pouvant être observée dans le cadre du mode de mesure choisi,
- "propriété spécifiquement dépendante" d'un paramètre : le fait que la propriété dépende du paramètre et de lui seul.

L'invention concerne un procédé de sélection d'agents, dits agents à tester, susceptibles d'être biologiquement actifs vis-à-vis d'un système cellulaire vivant en induisant un changement d'état biochimique d'un signal cellulaire à au moins deux états différents de la membrane cellulaire d'au moins une cellule vivante du système cellulaire vivant, dite cellule sensible, par utilisation d'un phénomène de couplage de luminescence entre au moins deux groupements photocomplémentaires, susceptible de générer et/ou de modifier au moins une émission lumineuse, dont au moins une propriété mesurable est spécifiquement dépendante de la distance séparant les groupements photocomplémentaires et est modifiée lorsque le signal cellulaire change d'état,
caractérisé en ce que :

- on prépare un système cellulaire vivant contenant au moins une cellule sensible,
- on introduit dans le système cellulaire ou on fait produire par le système cellulaire au moins un agent, dit agent intermédiaire marqué, choisi pour que, dans un premier état du signal cellulaire, cet agent intermédiaire marqué soit spécifiquement recruté à la membrane cellulaire d'au moins une cellule sensible, et pour que dans un deuxième état du signal cellulaire, cet agent intermédiaire marqué ne soit pas spécifiquement recruté par une membrane cellulaire de cellule sensible, ledit agent intermédiaire marqué étant une protéine naturelle marquée avec au moins un des groupements photocomplémentaires, dit premier luminophore, dans des conditions propres à préserver au moins sensiblement les propriétés du système cellulaire vivant vis-à-vis du signal cellulaire, cette protéine naturelle étant choisie parmi :

  - une protéine de transduction intracellulaire spécifique de l'état d'au moins un récepteur membranaire susceptible d'être activé par au moins un des agents à tester, ledit signal cellulaire étant un signal de transduction intracellulaire dont le premier état correspond au recrutement spécifique de la protéine de transduction à la membrane cellulaire, la protéine de transduction et le premier luminophore étant choisis pour que la protéine de transduction marquée présente au moins les fonctionnalités et le comportement de la protéine de transduction dans l'expression du signal de transduction intracellulaire par la(les) cellule(s) sensible(s),
  - une protéine intégrale de la membrane plasmique dont un fragment peut être libéré sous l'action de protéases,
  - une protéine intracellulaire qui peut être transloquée vers la membrane d'organites intracellulaires,

- on introduit dans le système cellulaire vivant au moins une composition d'au moins un composé, dit agent membranaire de couplage, apte à s'associer à une membrane cellulaire de cellule sensible et marqué avec au moins un groupement photocomplémentaire, dit deuxième luminophore, distinct du premier luminophore, et de telle sorte que les propriétés du système cellulaire vivant vis-à-vis du signal cellulaire soient au moins sensiblement préservées et qu'au moins une propriété mesurable de l'émission lumineuse résultant du couplage de luminescence entre le premier et le deuxième luminophore soit sensiblement modifiée lorsque le signal cellulaire change d'état ; ledit agent membranaire de couplage comprenant un ligand lipophile choisi parmi les phospholipides, les acides gras, les glycérides, le cholestérol, les céramides, les amines grasses, les agents luminophores lipophiles ou les dérivés lipophiles de ces composés ;
- on met le système cellulaire vivant ainsi préparé en présence d'une quantité de l'un des agents à tester, dit agent en test, on mesure au moins ladite propriété mesurable, et on détermine l'activité biologique de cette quantité de l'agent en test en fonction du résultat de cette mesure.

L'invention part ainsi du constat selon lequel de nombreux mécanismes d'activation ou d'inhibition cellulaire correspondent à des signaux biocellulaires dont la localisation, la durée et le fonctionnement ne sont pas élucidés, malgré de nombreuses études, en raison de limitations technologiques actuelles, mais qui en fait sont liés à des évènements se produisant à la membrane cellulaire et qui correspondent ou peuvent correspondre à un recrutement ou non à la membrane cellulaire d'un agent spécifique, en quantités importantes ou faibles, à l'intérieur ou à l'extérieur des cellules.

Par membrane cellulaire, on désigne indifféremment la membrane plasmique et d'autres systèmes membranaires de la cellule, tels que membrane mitochondriale, membrane golgienne, membrane du réticulum endoplamique, ou encore membrane de diverses vésicules du cytosol.

Ces évènements membranaires qui modifient l'état de la membrane cellulaire peuvent en particulier être de plusieurs natures.

Il peut s'agir tout d'abord purement et simplement du recrutement d'un agent à la membrane cellulaire. Cet agent peut alors servir d'agent intermédiaire marqué. Tel est le cas par exemple lors des mécanismes intracellulaires d'activation d'un signal de transduction intracellulaire.

Il peut s'agir aussi d'une activité enzymatique produite par la membrane cellulaire : activité kinase ou phosphatase (en général intracellulaire) ou activité protéase.

Il peut s'agir encore d'une transconformation d'une protéine membranaire (par exemple dans le cas d'un récepteur activé par un ligand).

Il peut s'agir aussi d'une interaction protéine-protéine ou protéine-lipide se produisant à la membrane cellulaire.

Il peut s'agir aussi d'une libération par clivage protéolytique d'au moins une partie d'une protéine intégrale de la membrane cellulaire, sous l'action de protéases particulières en réponse à un signal bien particulier.

A titre de mécanismes intracellulaires et de signaux cellulaires intracellulaires pouvant être impliqués dans un procédé selon l'invention, on peut citer plusieurs classes de récepteurs membranaires qui induisent un tel signal cellulaire : les récepteurs couplés aux protéines G ; les récepteurs à activité enzymatique de type tyrosine et sérine/thréonine kinases, ou tyrosine phosphatases ; les récepteurs sans activité enzymatique intrinsèque tels que les récepteurs aux cytokines associés aux protéines JAK ; les récepteurs à domaine TIR ou DD ; les récepteurs aux antigènes.

A titre de mécanismes extracellulaires et de signaux cellulaires extracellulaires, on peut citer des protéines intégrales de la membrane plasmique, notamment les enzymes de la famille des ADAM ou encore la protéine prion.

Les inventeurs ont ainsi déterminé d'une part, qu'il est possible d'incorporer dans la membrane cellulaire des agents membranaires de couplage, marqués par des groupements fluorescents, qui peuvent bénéficier de la dynamique latérale de la membrane cellulaire (en étant eux-mêmes soumis à cette dynamique) pour se répartir de façon homogène au sein de celle-ci et en une quantité qui simultanément soit assez faible pour ne pas affecter les propriétés fonctionnelles de la membrane cellulaire et de la cellule vivante, mais soit assez importante pour assurer la réalisation du couplage de luminescence lorsque l'agent intermédiaire marqué est recruté en tout endroit de la membrane cellulaire, quel que soit le site spécifique de translocation à la membrane cellulaire de cet agent intermédiaire marqué, et alors même que ce site n'est pas formé de l'un desdits agents membranaires de couplage.

Ainsi, il est à noter que dans un procédé de l'invention, et contrairement à tous les procédés antérieurs décrits, le couplage de luminescence intervient entre l'agent intermédiaire marqué et un agent (agent membranaire de couplage) qui ne correspond pas à l'agent de la membrane cellulaire spécifiquement reconnu (protéine, agent de reconnaissance...) par l'agent intermédiaire marqué lors de son recrutement à la membrane cellulaire ni au récepteur membranaire responsable dudit signal dont le changement d'état est induit par l'agent à tester. Mais les inventeurs ont constaté que grâce au fait que l'invention met à profit la structure de mosaïque fluide de la membrane cellulaire, statistiquement, il se trouve toujours un agent membranaire de couplage à proximité de l'agent intermédiaire marqué recruté à la membrane cellulaire, et à une distance qui en pratique correspond à l'obtention du phénomène de couplage de luminescence tel que le FRET. On sait en effet qu'une émission de luminescence spécifique par couplage de luminescence se produit ou est modifiée lorsque :

- (a) deux groupements photocomplémentaires sont proches dans l'espace (notamment de 1 à 10 nm) d'une distance inférieure ou égale à une valeur seuil prédéterminée (2 Ro, où Ro est le rayon de Forster dans le cas du FRET),
- (b) le spectre d'émission de l'un des groupements photocomplémentaires, dit donneur, et le spectre d'absorption de l'autre groupement photocomplémentaire, dit accepteur, se recouvrent au moins en partie (Lakey et al 1991, J. Mol. Biol., 1218, pp 639-653).

Egalement, les inventeurs ont mis en évidence qu'il existe des couples de luminophores compatibles d'une part, avec les agents membranaires (phospholipides), d'autre part, avec les agents intermédiaires selon l'invention. En particulier, il a été trouvé des lipides pouvant porter un composé fluorescent compatible avec les membranes cellulaires naturelles (pouvant faire office d'agents membranaires de couplage) et réalisant un couplage de fluorescence par FRET avec une GFP et notamment avec la GFP naturelle.

Il est ainsi important de noter que l'invention permet un criblage d'agents bioactifs sur des cellules vivantes. Ce criblage produit des résultats qui, par comparaison avec des méthodes similaires déjà connues, sont de fiabilité bien meilleure.

Ceci s'explique grâce à l'utilisation de cellules sensibles très comparables aux mêmes cellules dans un état naturel, aussi bien de par leur comportement que de par leur structure. En effet, l'invention fait intervenir, tout d'abord un agent intermédiaire qui n'est autre qu'une protéine naturelle, habituellement présente dans ce type de cellule (protéines de transduction, protéines intégrales et clivables de la membrane plasmique, protéines pouvant être transloquées à la membrane). Il est peu probable qu'un simple marquage d'une telle protéine, réalisé avec les techniques actuelles, puisse perturber significativement le comportement de la cellule, à l'inverse des anticorps utilisés dans certaines méthodes. L'invention fait également intervenir un agent membranaire de couplage qui, comme déjà mentionné, peut avantageusement être une sonde lipidique membranaire (par exemple la sonde DiI), capable de s'insérer uniformément sur l'ensemble de la membrane entre les phospholipides sans pour autant perturber la structure de cette membrane.

La fiabilité d'un criblage selon l'invention s'explique également par un résultat d'obtention et d'interprétration extrêmement simples. En effet, l'invention exploite avantageusement la translocation de certains constituants cellulaires (protéines de transduction, protéines intégrales et clivables de la membrane plasmique, protéines pouvant être transloquées à la membrane vers un système membranaire particulier ou en provenance de celui-ci, en réponse à un certain stimulus extracellulaire. L'invention exploite de façon nouvelle et inventive ce phénomène de translocation protéique particulier, en proposant une détection du phénomène de couplage de fluorescence uniquement au niveau de compartiments cellulaires particuliers (systèmes membranaires bien définis et facilement identifiables en microscopie) favorables à une analyse globale par spectroscopie simple et rapide ou à une observation en microscopie de fluorescence.

Avantageusement et selon l'invention, les premier et deuxième luminophores sont choisis pour réaliser un couplage de luminescence par transfert d'énergie par résonance, notamment par transfert d'énergie de fluorescence par résonance FRET.

Avantageusement et selon l'invention, l'un au moins des premier et deuxième luminophores est choisi parmi :

- une molécule susceptible d'absorber la lumière émise par une protéine fluorescente,
- une protéine fluorescente naturelle ou une protéine fluorescente mutée, par addition, délétion ou substitution d'un ou plusieurs acides aminés d'une protéine fluorescente naturelle, cette protéine fluorescente mutée conservant la propriété de fluorescence,
- une protéine bioluminescente naturelle ou mutée, notamment de type luciférase,
- un fragment fluorescent issu d'une protéine fluorescente naturelle ou d'une protéine fluorescente mutée adapté pour présenter la propriété de fluorescence de la protéine fluorescente dont il est issu,
- la fluorescéine, la rhodamine, le Bodipy®, la cyanine, le Nile Red®, le Texas Red®, les chélates de terres rares, les indo et carbocyanines, le diphénylhexatriène, les dérivés aromatiques luminescents.

Avantageusement et selon l'invention, l'un au moins des premier et deuxième luminophores est une protéine autofluorescente naturelle de cnidaire, notamment la protéine fluorescente verte GFP de la méduse *Aequorea victoria*.

Selon l'invention, on utilise un agent membranaire de couplage comprenant un ligand lipophile choisi parmi les phospholipides, les acides gras, les glycérides, le cholestérol, les céramides, les amines grasses, les agents luminophores lipophiles ou les agents dérivés de ces composés. Ces ligands lipophiles peuvent en effet être marqués par un deuxième luminophore. Le deuxième luminophore peut lui-même constituer le ligand lipophile et donc l'agent membranaire de couplage.

Selon un premier aspect de l'invention, les agents à tester sont des agents susceptibles d'être actifs sur des récepteurs membranaires, on choisit alors, en tant qu'agent intermédiaire marqué, une protéine de transduction intracellulaire spécifique de l'état desdits récepteurs membranaires, ledit signal cellulaire étant un signal de transduction intracellulaire dont le premier état correspond au recrutement spécifique de la protéine de transduction à la membrane cellulaire. L'agent intermédiaire marqué est alors formé de la protéine de transduction marquée par le premier luminophore, la protéine de transduction et le premier luminophore étant choisis pour que la protéine de transduction marquée présente au moins les fonctionnalités et le comportement de la protéine de transduction dans l'expression du signal de transduction intracellulaire par la(les) cellule(s) sensible(s).

En particulier, le premier luminophore est adapté pour préserver au moins sensiblement la réponse et la sensibilité de la(des) cellule(s) sensible(s) vis-à-vis de la protéine de transduction.

Avantageusement et selon l'invention, on réalise le marquage de la protéine de transduction par voie génétique en faisant exprimer par au moins une cellule sensible du système cellulaire vivant, une protéine de fusion formant la protéine de transduction marquée.

Avantageusement et selon l'invention, on choisit la protéine de transduction parmi :

- les protéines contenant des domaines (notamment les domaines PH ou FYVE) interagissant avec des lipides membranaires,
- les protéines s'associant à la membrane plasmique par ancrage lipidique, notamment de type N-myristoylation, S-palmrtoylation, S-prénylation,
- les protéines associées directement ou indirectement aux récepteurs couplés aux protéines G,
- les protéines associées directement ou indirectement aux récepteurs à activité enzymatique tyrosine kinase intrinsèque,
- les protéines STAT,
- les protéines associées directement ou indirectement aux récepteurs à activité enzymatique sérine/thréonine kinase intrinsèque,
- les protéines associées directement ou indirectement aux récepteurs à activité enzymatique tyrosine phosphatase intrinsèque,
- les protéines JAK associées aux récepteurs reconnaissant des cytokines correspondantes,
- les protéines associées directement ou indirectement aux récepteurs à domaine TIR ou DD,
- les protéines associées aux récepteurs reconnaissant des antigènes.

Plus particulièrement, les tableaux 1 à 8 suivants donnent des exemples de protéines de transduction présentes dans l'espèce humaine et pouvant être marquées et utilisées à titre d'agent intermédiaire marqué dans un procédé selon l'invention.

TABLEAU 1

| PROTEINES A DOMAINE PH | |
|---|---|
| CENTAURINES | CENTAURINE BETA2, CENTAURINE ALPHA2 |
| DYNAMINES | DYNAMINE 2, DYNAMINE 1 |
| FACTEURS D'ECHANGE DES NUCLEOTIDES GUANYLIQUES (dits GEF pour « GUANINE NUCLEOTIDE EXCHANGE FACTORS ») | GEF STIMULE PAR APC, GEF-H1 (PCNA P40), LSC, P115-RHOGEF, RHOGEF NON-OCOGENIQUE, RHO/RAC GEF (PROTEINE DE LA DYSPLASIE FACIOGENITALE), RALGPS1A, RAS-GRF1 (CDC25), PROTEINE ACTIVATRICE DE TYPE RASGAP 1, PROTEINE ACTIVATRICE DE TYPE RASGAP 2, TIAM1, TIAM2, ARF-GEP(100), TRIO, KALIRINE, GRP1, CYTOHESINE 1, CYTOHESINE 2 (ARNO), CYTOHESINE 3 (ARNO3), CYTOHESINE 4, DBL, LARG, VAV, VAV-2, VAV-3, COLLYBISTINE, ALPHA PIX, BETA PIX, P50COOL-1 |
| HOMOLOGUES DE LA PROTEINE « SON OF SEVENLESS » (dite SOS) | SOS-1, SOS-2 |
| KINASES DE TYPE ARK | BETA-ARK-1 (GRK2), BETA-ARK-2 (GRK3) |
| ONCOGENES/PROTO-ONCOGENES | DBL , ONCOGENE LBC (P47), P60TIM |
| PHOSPHOLIPASES C (PLC) | PLC-DELTA-1, PLC-GAMMA-1, PLC-GAMMA-2 |
| PHOSPHOLIPASES D (PLD) | PLD1, PLD2 |
| PLECKSTRINES | PLECKSTRINE, PLECKSTRINE 2, HOMOLOGUE DE LA PLECKSTRINE 2 |
| PRODUITS DES GENES KIAA | KIAA0006, KIAA0281, KIAA0351, KIAA0380, KIAA0400, KIAA0424, KIAA0571, KIAA0619, KIAA0763, KIAA0793, KIAA0902, KIAA0969, KIAA1099 |
| PROTEINES ADAPTATRICES | APPL, DAPP1, LNK, BAM32 (DAPP1), INTERSECTINE, CNK1 |
| PROTEINES ASSOCIEES A LA KINASE DE SRK | RA70, SKAP55 |

(suite)

| PROTEINES A DOMAINE PH | |
|---|---|
| PROTEINES CONTENANT UN DOMAINE PH EN TANDEM | TAPP1, TAPP2 |
| PROTEINES CONTENANT UN DOMAINE SH2 | PROTEINE DE SIGNAL SH2-B ALPHA, PROTEINE DE SIGNAL SH2-B BETA, PROTEINE DE SIGNAL SH2-B GAMMA |
| PROTEINES DE LA MOELLE OSSEUSE (dites BM pour « BONE MARROW ») | BM046, BM024 |
| PROTEINES DE LA SUPERFAMILLE DES KINESINES | KIF1B, KIF1A, KLP, KPL1, APS |
| PROTEINES DE TYPE « GRB » | GRB7, GRB 10, GRB 14 |
| PROTEINES KINASES C (PKCs) | PKC MU, PKC NU |
| PROTEINES KINASES (AUTRES) | PROTEINE LIANT LE CDC42 BETA, KINASE DE RHO, P160ROCK, MAGUIN-1 |
| PROTEINES LIANT L'ACTINE | ANILLINE, PROTEINE ASSOCIEE AUX FILAMENTS D'ACTINE |
| PROTEINES LIANT LE PHOSPHOINOSITOL 3-PHOSPHATE (dites PIP3-BP pour « PHOSPHOINOSITOL 3-PHOSPHATE BINDING PROTEINS ») | PIP3-BP-1, PIP3-BP-2 |
| PROTEINES LIANT LES PROTEINES DE TYPE « GRB » | GAB1, GAB2, PROTEINE ADAPTATRICE ASSOCIEE A GRB10 (GRB-IR), PROTEINE ADAPTATRICE ASSOCIEE A GRB14, PROTEINE ADAPTATRICE ASSOCIEE A GRB7 (B47) |
| PROTEINES LIANT LES « GAP » | P62DOK, P22DOK(DEL) |
| PROTEINES LIANT LES ANTIGENES (ABP) | GOODPASTURE ABP, D26 |
| PROTEINES SERINE-THREONINE KINASES | PKB (C-AKT), PKB BETA, PKB GAMMA, PDK1 |
| PROTEINES SIMILAIRES DE L'OSBP | PROTEINE SIMILAIRE DE L'OSBP 1, PROTEINE SIMILAIRE DE L'OSBP 4, PROTEINE SIMILAIRE DE L'OSBP 6, PROTEINE SIMILAIRE DE L'OSBP 7 |
| PROTEINES TYROSINE KINASES | BMX (KINASE DE LA MOELLE OSSEUSE LIEE A L'X), BTK (TYROSINE KINASE DE L'AGAMMAGLOBULINEMIE DE TYPE BRUTON, ITK, LYK, TEC |
| PROTEINS ACTIVANT LES GTPASES (dites GAP pour « GTPASE-ACTIVATING PROTEINS) | GAP 1 (PROTEINE LIANT L'INS P4), GAP 2 (GAP1M), NGAP, P120GAP (RASGAP), OLIGOPHRENINE 1, PROTEINE DE TYPE OLIGOPHRENINE-1 |
| PROTEINS PEPP | PEPP1, PEPP2, PEPP3 |
| SPECTRINES | SPECTRINE BETA5, SPECTRINE BETA4 SIGMA1, SPECTRINE BETA4 SIGMA3, SPECTRINE BETA3, SPTBN1, SPECTRINE BETA4 |
| SUBSTRATS DES RECEPTEURS DE L'INSULINE (dits IRS pour « INSULIN RECEPTOR SUBSTRATES ») | IRS-1, IRS-2, IRS-4 |
| SYNTROPHINES | SYNTROPHINE ALPHA1 (DJ1187J4.5), SYNTROPHINE BETA1, SYNTROPHINE BETA2, SYNTROPHINE 4, SYNTROPHINE 5 |

(suite)

| PROTEINES A DOMAINE PH | |
|---|---|
| AUTRES PROTEINES A DOMAINES PH | CDEP, DUET, HAP1 (DUO), MYOSINE X, PHR1, PROTEINE ADAPTATRICE ASSOCIEE A SRC, SWAP-70, FAPP1, DEF-6, JBP, BRDG1, GAB2, GRB7V, SUBSTRAT DE LA KINASE BRK, PKD2 |

TABLEAU 2

| PROTEINES SANS DOMAINE PH MAIS A DOMAINE FYVE, C1 OU C2 | |
|---|---|
| EFFECTEURS DE RAB | RABIP4, RABENOSYNE-5 |
| PROTEINES ASSOCIEES A SMAD | SARA, HGS (HRS) |
| PROTEINES CONTENANT UN DOUBLE DOMAINE FYVE | DFCP1, TAFF1 |
| PROTEINES PHOSPHATASES A DOUBLE SPECIFICITE | FYVE-DSP2, FYVE-DSP1A, FYVE-DSP1B, FYVE-DSP1C |
| PROTEINES KINASES C (PKC) | PKC ALPHA, PKC BETA1, PKC BETA2, PKC GAMMA, PKC DELTA, PKC EPSILON, PKC ETA, PKC THETA, PKC ZETA, PKC IOTA |
| AUTRES PROTEINES A DOMAINE FYVE | EIP1, KIAA0305, ANKHZN |
| AUTRES PROTEINES A DOMAINE C1 OU C2 | N-CHIMAERINE ALPHA1, N-CHIMAERINE ALPHA2, N-CHIMAERINE BETA1, N-CHIMAERINE BETA2, NEDD-4, PERFORINE 1 |

TABLEAU 3

| PROTEINES SANS DOMAINE PH OU FYVE OU C1 OU C2 MAIS ASSOCIEES A LA MEMBRANE PLASMIQUE PAR ANCRAGE LIPIDIQUE | |
|---|---|
| PROTEINES N-MYRISTOYLEES | MARCKS, MRP, SYNTHASE DE L'OXYDE NITRIQUE (ENOS), RECOVERINE, PP60SRC |
| PROTEINES S-PALMITOYLEES | GS ALPHA, GQ ALPHA, CHIC2 |
| PROTEINES S-PRENYLEES | DOCK180, RHO A |

TABLEAU 4

| PROTEINES SANS DOMAINES PH OU FYVE OU C1 OU C2, SANS ANCRAGE MEMBRANAIRE MAIS ASSOCIEES DIRECTEMENT A DES RECEPTEURS MEMBRANAIRES | |
|---|---|
| PROTEINES ASSOCIEES AUX RECEPTEURS COUPLES AUX PROTEINES G | BETA ARRESTINE 1, BETA ARRESTINE 2, GRK1, GRK4, GRK5, GRK6, DISHEVELLED, GBP |
| PROTEINES ASSOCIEES AUX RECEPTEURS DES ANTIGENES | LCK, ZAP-70 (SYK), SHP-1 |
| PROTEINES CONTENANT DES DOMAINES PTB | FRS2 ALPHA, FRS2 BETA |
| PROTEINES CONTENANT DES DOMAINES SH2 | GRB2, SHC, P85, SOCS-3, CBL |
| PROTEINES CONTENANTS DES DD (pour « DEATH DOMAIN ») | TRADD, RIP, RAIDD, MYD88 |
| PROTEINES SMAD (CO-MODULATRICES DE LA TRANSCRIPTION) | SMAD1, SMAD2, SMAD3, SMAD5, SMAD8 |

(suite)

| PROTEINES SANS DOMAINES PH OU FYVE OU C1 OU C2, SANS ANCRAGE MEMBRANAIRE MAIS ASSOCIEES DIRECTEMENT A DES RECEPTEURS MEMBRANAIRES | |
|---|---|
| PROTEINES STAT (ACTIVATRICES DE LA TRANSCRIPTION) | STAT1, STAT2, STAT3, STAT5 |
| PROTEINES TRAF | TRAF1, TRAF2, TRAF3, TRAF5, TRAF6 |
| PROTEINES TYROSINES PHOSPHATASES | PTP1B |
| PROTEINES A DOMAINE PDZ | MUPP1, NHERF, RGS12, HOMER |

TABLEAU 5

| PROTEINES SANS DOMAINES PH OU FYVE OU C1 OU C2, SANS ANCRAGE MEMBRANAIRE MAIS ASSOCIEES INDIRECTEMENT AVEC DES RECEPTEURS MEMBRANAIRES | |
|---|---|
| CYSTEINES PROTEASES | CASPASE-8 (FLICE), CASPASE-10, CASPASE-2 |
| PHOSPHOLIPIDES KINASES | P110 |
| PHOSPHOLIPIDES PHOSPHATASES | PTEN, SHIP |
| PROTEINES CONTENANT DES DD (pour « DEATH DOMAIN ») | IRAK, IRAK2, IRAKM, MORT1 (FADD) |
| PROTEINES D'ECHAFFAUDAGE | KSR |
| PROTEINES KINASES A DOUBLE SPECIFICITE | MEK1, MEK2 |
| PROTEINES SERINE-THREONINE KINASES | RAF1, A-RAF, B-RAF, ERK1, ERK2, TAK1 |
| PROTEINES TYROSINE KINASES | PYK2 |
| PROTEINES ACTIVANT LES GTPASES (dites GAP pour « GTPASE-ACTIVATING PROTEINS) | RGS8 |
| AUTRES | TAB1, TAB2, FLIP |

TABLEAU 6

| PROTEINES ASSOCIEES A LA MEMBRANE PLASMIQUE MAIS SELON UN MECANISME DIFFERENT DE CEUX DECRITS DANS LES TABLEAUX 1 A 5 | |
|---|---|
| FACTEURS D'ADP-RYBOSYLATION | ARF5, ARF6 |
| TRANSPORTEURS DE GLUCOSE | GLUT-4 |
| KINASE | PAK1, PAK2 |
| PROTEINE ASSOCIEE A LA MYELINE | MBP |
| PROTEINES A DOMAINE PX | P40PHOX, P47PHOX |

TABLEAU 7

| PROTEINES INTEGRALES DE LA MEMBRANE CONTENANT DES PEPTIDES LIBERES PAR PROTEOLYSE DANS LE MILIEU EXTRA- OU INTRACELLULAIRE | |
|---|---|
| FACTEURS DE COAGULATION | PROTHROMBINE, FACTOR V, FACTOR VIII |
| SUBSTRATS DES METALLOPROTEASES DE TYPE ADAM 10 | COLLAGENE DE TYPE IV, EPHRINE-A2 |
| SUBSTRATS DES METALLOPROTEASES DE TYPE ADAM 12 | ALPHA2-MACROGLOBULINE |

(suite)

| PROTEINES INTEGRALES DE LA MEMBRANE CONTENANT DES PEPTIDES LIBERES PAR PROTEOLYSE DANS LE MILIEU EXTRA- OU INTRACELLULAIRE | |
|---|---|
| SUBSTRATS DES METALLOPROTEASES DE TYPE TACE | TNF ALPHA, TNF DELTA, TGF ALPHA, L-SELECTINE, TNF-RI, TNF-RII, APP, IL-1RII, IL-6R, ERBB4, ACE, TRANCE, NOTCH, HB-EGF |
| AUTRES | PRESENILINE, PROTEINE PRION, PRO-NEUREGULINE, FASL |

TABLEAU 8

| PROTEINES ASSOCIEES A DES MEMBRANES INTRACELLULAIRES AUTRES QUE LA MEMBRANE PLASMIQUE | |
|---|---|
| PROTEINES TRANSLOQUEES VERS LA MEMBRANE MITOCHONDRIALE | BAX, BCL-X(L), BAD, BID |
| PROTEINES A DOMAINE PX | CISK, SNX3, SNX7, RGS-PX |

Selon un deuxième aspect de l'invention, l'agent intermédiaire n'est pas une protéine de transduction, mais est une protéine intégrale de la membrane plasmique dont un fragment peut être libéré sous l'action de protéases. Plus particulièrement, le tableau 7 donne des exemples de telles protéines présentes dans l'espèce humaine.

Selon un dernier aspect de l'invention, l'agent intermédiaire est une protéine intracellulaire qui peut être transloquée vers la membrane d'organites intracellulaires, notamment les mitochondries, le Golgi, le réticulum endoplasmique. Plus particulièrement, le tableau 8 donne des exemples de telles protéines présentes dans l'espèce humaine.

Quoiqu'il en soit, il est à noter que dans un procédé selon l'invention, l'agent intermédiaire marqué est généralement distinct de l'agent en test.

Avantageusement et selon l'invention, le système cellulaire vivant comprend au moins une cellule sensible eucaryote vivante. Il peut s'agir de préférence d'une ou plusieurs cellule(s) de mammifère, notamment humaine(s). Le système cellulaire vivant peut être un système isolé d'un organisme, et comprend un milieu de culture artificiel incorporant au moins une cellule sensible vivante, en général une pluralité de cellules sensibles vivantes. Le système cellulaire vivant peut ainsi être au moins une partie d'un organisme pluricellulaire vivant, c'est-à-dire une plante ou une partie de plante, ou un animal, ou une partie d'un animal non humain ou un élément isolé du corps humain (biopsies, échantillons sanguins...).

L'invention s'étend à un système cellulaire vivant, à l'exception d'un embryon humain, d'un corps humain ou d'un élément non isolé d'un corps humain, modifié pour la mise en oeuvre d'un procédé selon l'invention.

L'invention concerne ainsi un système cellulaire vivant à l'exception d'un embryon humain, d'un corps humain ou d'un élément non isolé d'un corps humain, comprenant au moins une cellule vivante dont la membrane cellulaire incorpore au moins un composé, dit agent membranaire de couplage, marqué avec au moins un groupement, dit deuxième luminophore, photocomplémentaire d'au moins un autre groupement photocomplémentaire, dit premier luminophore, distinct du deuxième luminophore, dans un phénomène de couplage de luminescence susceptible de générer et/ou de modifier au moins une émission lumineuse dont au moins une propriété mesurable est spécifiquement dépendante de la distance séparant les groupements photocomplémentaires, l'agent membranaire de couplage étant associé à la membrane cellulaire en une quantité adaptée pour au moins sensiblement préserver les propriétés du système cellulaire vivant vis-à-vis d'un signal cellulaire à au moins deux états différents de la membrane cellulaire d'au moins une cellule vivante, dite cellule sensible, du système cellulaire vivant, et pour qu'au moins une propriété mesurable de l'émission lumineuse résultant du couplage de luminescence soit sensiblement modifiée lorsque le signal cellulaire change d'état, le premier luminophore marquant au moins un agent, dit agent intermédiaire marqué, adapté pour être spécifiquement recruté à la membrane cellulaire d'au moins une cellule sensible dans un premier état du signal cellulaire et pour ne pas être spécifiquement recruté par une membrane cellulaire de cellule sensible dans un deuxième état du signal cellulaire, ledit agent intermédiaire marqué étant une protéine naturelle marquée avec le premier luminophore, cette protéine naturelle étant choisie parmi :

- une protéine de transduction intracellulaire spécifique de l'état d'au moins un récepteur membranaire susceptible d'être activé par au moins un des agents à tester, ledit signal cellulaire étant un signal de transduction intracellulaire dont le premier état correspond au recrutement spécifique de la protéine de transduction à la membrane cellulaire, la protéine de transduction et le premier luminophore étant choisis pour que la protéine de transduction marquée présente au moins les fonctionnalités et le comportement de la protéine de transduction dans l'expression du signal de transduction intracellulaire par la(les) cellule(s) sensible(s),

- protéine intégrale de la membrane plasmique dont un fragment peut être libéré sous l'action de protéases,
- une protéine intracellulaire qui peut être transloquée vers la membrane d'organites intracellulaires, l'agent membranaire de couplage ne correspondant pas à l'agent de la membrane cellulaire spécifiquement reconnu par un agent intermédiaire marqué lors de son recrutement à la membrane cellulaire et comprenant un ligand lipophile choisi parmi les phospholipides, les acides gras, les glycérides, le cholestérol, les céramides, les amines grasses, les agents luminophores lipophiles ou les dérivés lipophiles de ces composés.

L'agent membranaire de couplage et l'agent intermédiaire marqué sont conformes aux caractéristiques mentionnées ci-dessus en référence au procédé de sélection de l'invention. En particulier, avantageusement et selon l'invention, au moins une cellule sensible, dont la membrane cellulaire incorpore au moins un agent membranaire de couplage, contient une séquence d'ADN comprenant un gène codant pour une protéine, dite protéine intermédiaire, choisie pour être spécifiquement recrutée à la membrane cellulaire de la(des) cellule(s) sensible(s) lorsque le signal cellulaire est dans un premier état, et pour ne pas être recrutée par la membrane cellulaire de la(des) cellule(s) sensible (s) lorsque le signal cellulaire est dans un deuxième état, fusionné avec au moins un gène codant pour un premier luminophore, la fusion de ces gènes étant telle que :

- lorsque le signal cellulaire est dans le premier état, une protéine de fusion, dite protéine intermédiaire marquée, formée de la protéine intermédiaire et du premier luminophore est exprimée par la(les) cellule(s) sensible(s) et recrutée à la membrane cellulaire, et fait office d'agent intermédiaire marqué,
- lorsque le signal cellulaire est dans le deuxième état, aucune protéine intermédiaire marquée n'est recrutée par une membrane cellulaire de cellule sensible.

Ladite protéine intermédiaire marquée peut être une protéine de transduction marquée.

Avantageusement, dans un système cellulaire vivant conforme à l'invention le premier et le deuxième luminophore sont choisis comme mentionné ci-dessus en référence au procédé de sélection de l'invention.

Avantageusement et selon l'invention, le système cellulaire comprend au moins une cellule sensible eucaryote vivante, notamment comme mentionné ci-dessus en référence au procédé de sélection de l'invention.

L'invention concerne aussi un procédé de sélection et un système cellulaire vivant caractérisés en combinaison par tout ou partie des caractéristiques mentionnées ci-dessus ou ci-après.

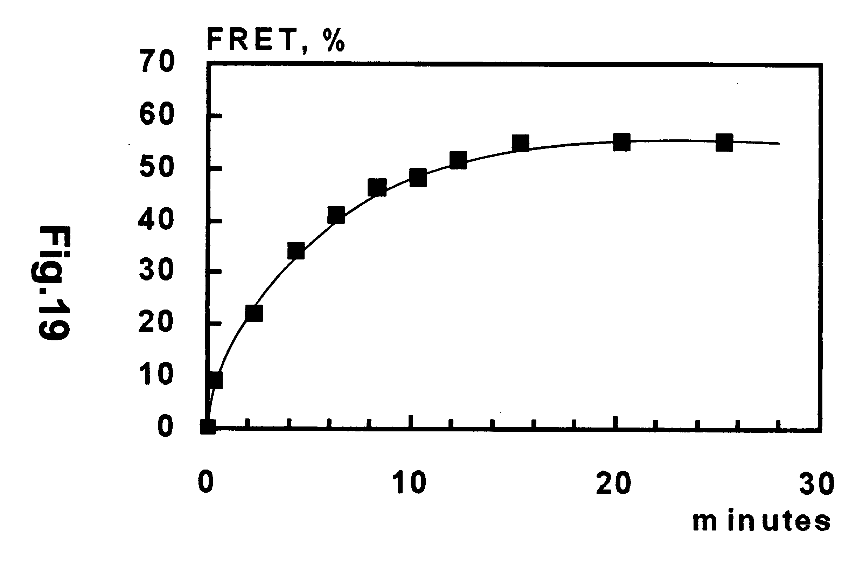

D'autres buts, caractéristiques et avantages de l'invention apparaissent à la lecture des exemples qui suivent. Les figures 1 à 19 illustrent des résultats obtenus dans les exemples :

- les figures 1, 6, 10 et 14 correspondent à des cartes de restriction de plasmides codant respectivement pour les protéines Cycle3-GFP-Akt, MOR-1-Cycle3-GFP et GFP-RafCAAX, NT-GFP-PKC,
- les figures 2, 3, 4, 9, 13, 16 et 17 représentent des spectres d'émission de fluorescence, avec ou sans correction des données,
- les figures 5 et 18 sont des représentations graphiques illustrants l'influence mutuelle de la Cycle3-GFP et de la sonde membranaire DiI, dans différentes conditions de test,
- les figures 7, 8, 11, 12, 15 sont des photographies de fluorescence permettant de visualiser la localisation intracellulaire des partenaires du phénomène de FRET,
- la figure 19 est une représentation graphique illustrant l'efficacité du transfert de fluorescence entre la Cycle3-GFP-PKCβII et la sonde membranaire DiI.

Exemple n° 1 : Couplage de Luminescence et Couples de Luminescence

COUPLAGE DE LUMINESCENCE

On nomme FRET (pour Fluorescence Resonance Energy Transfer), ou BRET (pour Bioluminescence Resonance Energy.Transfer), les techniques basées sur l'utilisation d'un mécanisme de transfert non radiatif de fluorescence par couplage dipolaire entre deux molécules appelées respectivement Donneur et Accepteur. Le FRET et le BRET sont souvent mis à profit pour déterminer précisément la distance entre les deux partenaires. Ces techniques s'appuient sur la théorie développée par Förster en 1948 (V.Th. Fôrster, (1948), Ann. Phys., 2, 54-75. Zwischenmolekular energiewanderung und Fluoreszenz) et tirent profit de la forte dépendance de l'efficacité du transfert (E) vis à vis de la distance entre les deux partenaires. En effet, l'efficacité du FRET, déterminée expérimentalement comme une variation d'intensité de fluorescence de l'Accepteur et/ou du Donneur, décroît très rapidement avec la distance (r) séparant le Donneur et l'Accepteur selon une fonction en $1/r^6$.

$$E = (I_0 - I) / I_0 = R_0{}^6 / (R_0{}^6 + r^6)$$

où, $I_0$ est l'intensité de fluorescence du Donneur lorsque le Donneur et l'Accepteur sont à une distance infinie, où I est l'intensité de fluorescence du Donneur lorsque le Donneur et l'Accepteur sont à une distance (r) et où $R_0$ est une grandeur caractéristique du couple Donneur/Accepteur et qui correspond à la distance pour laquelle l'efficacité de transfert de fluorescence est de 50% de sa valeur maximum. Au niveau membranaire, la détection d'un signal de FRET sera d'autant meilleure que la valeur de $R_0$ est élevée. Cette valeur, qui pourra théoriquement varier entre 15 et 120 Å, est calculée de façon théorique en utilisant la formule donnée par J.R. LAKOVICZ dans " Principles of Fluorescence Spectroscopy ", Seconde édition, Kluwer Academic/Plenum Publishers, 1999,13, 368-391 :

$$R_0 \text{ (en Å)} = 9,79 \; 10^3 . \; (K^2.n^{-4}.\phi_D.J)^{1/6}$$

Où, $K^2$ est le coefficient d'orientation des moments de transition d'émission du Donneur et d'absorption de l'Accepteur. La valeur de ce coefficient $K^2$ est égale à 2/3 lorsque le Donneur et l'Accepteur se distribuent de façon isotrope. n est l'indice de réfraction du milieu utilisé, $\phi_D$ est le rendement quantique de fluorescence du Donneur et J est l'intégrale de recouvrement des spectres d'émission du Donneur et d'absorption de l'Accepteur calculée comme suit :

$$J = \int F_D(\lambda) * \varepsilon_A(\lambda) * \lambda^4 \, d\lambda / \int F_D(\lambda) d\lambda$$

Où $F_D(\lambda)$ est la fluorescence du Donneur à la longueur d'onde $\lambda$ en nm, ou $\varepsilon_A(\lambda)$ est le coefficient d'extinction moléculaire exprimé en $M^{-1}.cm^{-1}$ à la longueur d'onde $\lambda$ en nm.

Dans cet exemple, différents couples Accepteur/Donneur ont été étudiés. Les propriétés spectroscopiques des partenaires (absorbance, coefficient d'extinction moléculaire, émission de fluorescence, rendement quantique, intégrales de recouvrement) ont été analysées et les valeurs des rayons de Fôrster ($R_0$), caractéristiques des différents couples de luminescence ont été calculées. Au sein de ces couples de luminescence, l'un des partenaires est soit une protéine fluorescente pour le FRET, ou bien une protéine bioluminescente pour le BRET, chacune manipulée avec les outils de la biologie moléculaire et produite dans la cellule vivante à partir d'un acide nucléique. Pour le FRET, il s'agira d'une Protéine Fluorescente Verte, dite «Green Fluorescent Protein» (GFP) ou certains de ses divers mutants. Pour le BRET, il s'agira d'une Luciférase.

L'autre partenaire est une molécule lipophile pouvant facilement être insérée de façon stable et quantitative dans la membrane plasmique de cellules vivantes.

Ce couple de luminescence Donneur/Accepteur est caractérisé par le fait que la valeur théorique de son $R_0$ est la plus élevée possible, ce qui signifie que :

1/ - dans ce couple Donneur/Accepteur, le rendement quantique de fluorescence du Donneur est le plus proche possible de 1,0 ;

2/ - dans ce couple Donneur/Accepteur, l'intégrale de recouvrement (J) doit être maximale entre le spectre d'émission du donneur et le spectre d'excitation de l'accepteur, ce qui signifie :

- dans ce couple Donneur/Accepteur, le coefficient d'extinction moléculaire de l'accepteur doit être le plus élevé possible ;
- dans ce couple Donneur/Accepteur, les longueurs d'ondes d'émission du Donneur et d'absorption de l'Accepteur doivent être les plus élevées possibles ;
- dans ce couple Donneur/Accepteur, les spectres d'émission de fluorescence du Donneur et de l'Accepteur doivent présenter un recouvrement spectral minimal ;
- dans ce couple Donneur/Accepteur, le coefficient d'extinction moléculaire de l'accepteur à la longueur d'onde d'excitation du donneur doit être le plus faible possible.

Par ailleurs, dans ce couple Donneur/Accepteur :

- la fluorescence du Donneur doit être la plus stable possible vis-à-vis de l'irradiation lumineuse ;
- la longueur d'onde d'excitation du Donneur doit être au moins supérieure à 360 nm et de préférence supérieure ou égale à 400 nm pour éviter l'autofluorescence des cellules ;
- l'Accepteur doit présenter un rendement quantique suffisant pour que la variation de sa fluorescence au cours du FRET soit aisément quantifiable.

<u>COUPLES DE LUMINESCENCE</u>

Plusieurs exemples concernant des couples Donneur/Accepteur d'intérêt majeur sont décrits ci-dessous :

<u>1 - Cycle3-GFP (Donneur) / Lipide fluorescent (Accepteur) dans le cas de FRET</u>

<u>La Cycle3-GFP</u>

La Cycle3-GFP est un mutant thermostable de la Protéine Fluorescente Verte (GFP), commercialisée par la société Invitrogen Life Technologies, Cergy Pontoise, France. C'est une protéine de 238 acides aminés qui peut être exprimée dans des cellules eucaryotes en conservant toutes ses propriétés de fluorescence. L'émission de la Cycle3-GFP se produit avec un maximum à 508 nm avec un rendement quantique très élevé de 79%, qui représente un avantage énorme par rapport à tous les autres mutants de la GFP. L'excitation de la Cycle3-GFP se produit entre 350 nm et 490 nm avec un maximum d'absorption à 398 nm. Le coefficient d'extinction moléculaire de la Cycle3-GFP à 398 nm est de 25000 $M^{-1}.cm^{-1}$. Le décalage important entre la longueur d'onde d'excitation ($\lambda_{exc}$ = 398 nm) et la longueur d'onde d'émission ($\lambda_{em}$ = 508 nm) permet une excellente séparation spectrale entre les spectres d'absorption de la Cycle3-GFP en tant que Donneur et de l'Accepteur lipidique, évitant ainsi l'excitation directe de l'Accepteur à la longueur d'onde d'excitation du Donneur. Enfin, la longueur d'onde d'excitation à 400 nm reste parfaitement supportée par les cellules vivantes, au contraire des illuminations plus énergétiques de l'ultraviolet nécessaires à l'excitation du mutant P4-3 (Y66H/Y145F, Protéine Fluorescente Bleue, ($\lambda_{exc}$ = 380 nm et rendement quantique de fluorescence de 20%)). Parmi les Donneurs possibles de fluorescence au sein du couple Donneur/Accepteur, le mutant W7 (Protéine Fluorescente Cyan) excité à 433 nm et analysé à 475 nm présente un déplacement de Stokes faible de 42 nm et un rendement quantique de fluorescence faible de 24%. Les mutants du type EYFP (Protéine Fluorescente Jaune) excités à 513 nm et analysé à 527 nm présente un déplacement de Stokes très faible de 14 nm et un rendement quantique de fluorescence faible de 20%. Les performances des mutants ECFP et EYFP sont donc largement insuffisantes. En dernier lieu, la longueur d'onde d'excitation de la Cycle3-GFP à 400 nm correspond à une bande d'émission intense des lampes à vapeur de mercure couramment utilisées avec les microscopes à fluorescence.

<u>Partenaires de la Cycle3-GFP</u>

Divers candidats lipidiques fluorescents, potentiels partenaires de FRET avec la Cycle3-GFP sont présentés dans le Tableau 9. Ils ont été analysés selon la qualité du recouvrement spectral avec le spectre d'émission de la Cycle3-GFP. La valeur du $R_0$ de ces différents candidats comme accepteur de FRET avec la Cycle3-GFP est donnée ainsi que la valeur du coefficient d'extinction moléculaire ($\varepsilon$, $M^{-1}.cm^{-1}$) à la longueur d'onde d'excitation de la Cycle3-GFP.
Se dégagent d'abord des composés dérivés de la rhodamine, commercialisés par Molecular Probes (Leiden, Pays-Bas), et dont le calcul du $R_0$ entre la Cycle3-GFP et le lipide fluorescent atteint des valeurs élevées, proches de 60 Å. Cependant, ces composés présentent un coefficient d'extinction moléculaire résiduel élevé (1800 à 5000 $M^{-1}.cm^{-1}$) à la longueur d'onde d'excitation de la Cycle3-GFP (400 nm).
Sont ensuite présentés des composés de la famille des cyanines, de la fluorescéine, des Bodipy®, AlexaFluor® et NileRed®. Parmi ces composés, certains présentent une valeur résiduelle de coefficient d'extinction moléculaire à 400 nm encore importante. Dans cette catégorie sont rangés des composés du type : AlexaFluor®546 ou Bodipy®TMR-X,SE commercialisés par Molecular Probes (Leiden, Pays-Bas) dont les valeurs de $P_0$ sont de 58,3 Å et $\varepsilon_{400\ nm}$ = 4264 $M^{-1}.cm^{-1}$ et 4422 $M^{-1}.cm^{-1}$.
Plus intéressants sont les composés dérivés de la fluorescéine, JOE, ou Bodipy®558 commercialisés par Molecular Probes (Leiden, Pays-Bas) dont les $R_0$ sont de 59,7 Å et de 61,2 Å et les coefficients d'extinction moléculaire à 400 nm de 1266 $M^{-1}.cm^{-1}$ et de 563 $M^{-1}.cm^{-1}$, respectivement.
A partir des données et des calculs présentés ci-dessus, la sonde lipophile la plus adaptée au FRET fait partie de la famille des cyanines, la dioctadécyl-1,1'-tétraméthyl-3,3,3',3'-indocarbocyanine (DiI ; Molecular Probes (Leiden, Pays-Bas, réf D-282)), pour laquelle le $R_0$ est de 67,4 Å et le coefficient d'extinction moléculaire résiduel à 400 nm de 245 $M^{-1}.cm^{-1}$.

TABLEAU 9

| Accepteur | Source Référence | Rayon de Förster Don/Acc $R_0$, en Å | $\lambda_{exc}$ Donneur $\varepsilon$ Acc à $\lambda_{exc}$ du Donneur | Propriétés |
|---|---|---|---|---|
| 6-JOE, SE succinimidyle carboxy-6-Dichloro-4',5'-diméthoxy-2',7'-Carboxylate de fluorescéine | Molecular Probes Leiden, Pays-Bas C-6171 | 59.7 | 400nm 1266 $M^{-1}.cm^{-1}$ | fluorophore greffable sur lipide ou protéine membranaires |
| Alexa Fluor® 546 Ester Succinimidylique de l'acide Alexa Fluor® 546 | Molecular Probes Leiden, Pays-Bas A-10237 | 58.3 | 400nm 4264 $M^{-1}.cm^{-1}$ | fluorophore greffable sur lipide ou protéine membranaires |
| BODIPY® TMR-X, SE Ester Succinimidylique du BODIPY® TMR-X | Molecular Probes Leiden, Pays-Bas D-6117 | 58.3 | 400nm 4422 $M^{-1}.cm^{-1}$ | fluorophore greffable sur lipide ou protéine membranaires |
| BODIPY®558/568, SE Ester Succinimidylique du BODIPY® 558/568 | Molecular Probes Leiden, Pays-Bas D-2219 | 61.2 | 400nm 563 $M^{-1}.cm^{-1}$ | fluorophore greffable sur lipide ou protéine membranaires |
| Cy3™ | Amersham Life Science, Inc | 64.4 | 400nm 254 $M^{-1}.cm^{-1}$ | fluorophore greffable sur lipide ou protéine membranaires |
| Dil ; DilC$_{18}$(3) Dioctadécyl-1,1'-tétraméthyl-3,3,3',3'-indocarbocyanine | Molecular Probes Leiden, Pays-Bas D-282 | 67.4 | 400nm 245 $M^{-1}.cm^{-1}$ | Cation Lipophile 0,05 < $\phi_f^{donneur}$ < 0,15 |
| nile red | Molecular Probes Leiden, Pays-Bas N-1142 | 58.0 | 400nm 216 $M^{-1}.cm^{-1}$ | Sonde lipophile Non fluorescent dans l'eau |
| R18 Octadécyle rhodamine B | Molecular Probes Leiden, Pays-Bas O-246 | 61.8 | 400nm 2317 $M^{-1}.cm^{-1}$ | Cation Lipophile |
| Lissamine™ Rhodamine B DHPE | Molecular Probes Leiden, Pays-Bas L-1392 | 54.1 | 400nm 1794 $M^{-1}.cm^{-1}$ | Phospholipide membranaire |

(suite)

| Accepteur | Source Référence | Rayon de Förster Don/Acc $R_0$, en Å | $\lambda_{exc}$ Donneur<br>ε Acc à $\lambda_{exc}$ du Donneur | Propriétés |
|---|---|---|---|---|
| Rhodamine Red™-X, SE<br>Ester Succinimidylique de la Rhodamine Red™-X | Molecular Probes<br>Leiden, Pays-Bas R-6160 | 60,1 | 400nm<br>3587 $M^{-1}.cm^{-1}$ | fluorophore greffable sur lipide ou protéine membranaires |
| TAMRA-X, SE<br>Ester Succinimidylique du TAMRA-X | Molecular Probes Leiden, Pays-Bas T-6105, C-2211, C-6123, C-1171 | 60,4 | 400nm<br>3669 $M^{-1}.cm^{-1}$ | fluorophore greffable sur lipide ou protéine membranaires |
| TRITC<br>Isothiocyanante de Tétraméthyle Rhodamine | Molecular Probes Leiden, Pays-Bas T-490, T1391 T-1481, T-1480 | 61,5 | 400nm<br>4667 $M^{-1}.cm^{-1}$ | fluorophore greffable sur lipide ou protéine membranaires |
| 5(6)-XRITC<br>Isothiocyanante de 5 (et 6)-X-Rhodamine | Molecular Probes Leiden, Pays-Bas X-491 | 43,2 | 400nm<br>4192 $M^{-1}.cm^{-1}$ | fluorophore greffable sur lipide ou protéine membranaires |

De plus, elle présente une affinité naturelle pour les membranes et permet le marquage membranaire de cellule par dispersion du produit dans le milieu de culture. Chargée positivement, elle aura une localisation privilégiée sur le feuillet interne de la membrane plasmique en réponse au potentiel transmembranaire. Par l'ensemble de ces propriétés, la sonde DiI est parfaitement adaptée à la mise en oeuvre de FRET sur cellules vivantes.

Par ailleurs, des sondes fluorescentes, analogues de phospholipides, sont intéressantes pour leur capacité à résider longtemps sur le feuillet externe de la membrane plasmique et représentent des candidats de choix pour étudier des translocations entre la membrane plasmique et le milieu extracellulaires. A ce critère, répond pleinement la sonde Lissamine™ Rhodamine DHPE ($R_0$ = 54,1 Å et $\varepsilon_{400\ nm}$ = 1794 $M^{-1}.cm^{-1}$). D'autres chromophores peuvent être avantageusement greffés sur des lipides naturellement présents dans la membrane par l'intermédiaire d'un groupement succinimidyle, par exemple :

TAMRA, SE ($R_0$ = 60,4 Å et $\varepsilon_{400\ nm}$ = 3669 $M^{-1}.cm^{-1}$), RhodamineRedX, SE ($R_0$ = 60,1 Å et $\varepsilon_{400\ nm}$ = 3587 $M^{-1}.cm^{-1}$), Cy3™ ($R_0$ = 64,4 Å et $\varepsilon_{400\ nm}$ = 254 $M^{-1}.cm^{-1}$), ou par l'intermédiaire d'un groupement isothiocyanate : TRITC ($R_0$ = 61,5 Å et $\varepsilon_{400\ nm}$ = 4667 $M^{-1}.cm^{-1}$).

### 2 - DsRed Protéine (Donneur) / Lipide fluorescent (Accepteur) dans le cas de FRET

### DsRed Protéine

Ces protéines fluorescentes «rouges» (dites «DsRed proteins ») ont été décrites pour la première fois en 1999 (Matz MM, Fradkov AF, Labas YA, Savitsky AP, Zaraisky AG, Markelov ML and Lukyanov SA, (1999), Nature Biotechnology, *17*, 969-973. Fluorescent Proteins from nonbioluminescent Anthozoa Species) dans des organismes associés aux récifs coralliens. Elles sont maintenant distribuées par Clontech Laboratories Inc., Palo Alto CA (USA). L'une de ces protéines, drFP583, présente un spectre d'excitation à 558 nm et un spectre d'émission de fluorescence à 583 nm. A ce titre, elle représente un candidat potentiel de premier choix comme Donneur de fluorescence dans des expériences de FRET. De plus, les longueurs d'ondes élevées d'absorption et d'émission de fluorescence laissent entrevoir des valeurs élevées de $R_0$ avec des sondes lipidiques membranaires du type Texas Red®. Cependant, le rendement quantique de la drFP583 décrite dans ce travail n'est que de 0,23.

Partenaires de la Protéine DsRed

Les avantages à l'utilisation des GFP « rouges » résident dans leurs longueurs d'onde d'émission de fluorescence largement déplacées vers le rouge, favorisant des valeurs de $R_0$ élevées. Cependant, en l'état actuel des connaissances, ces protéines semblent s'exprimer sous la forme de multimères dans des cellules ce qui représente un frein majeur à l'utilisation de ces protéines comme partenaire de FRET. De plus la cinétique de maturation de la DsRed à partir d'un analogue de GFP est lente et peut prendre plusieurs jours à température ambiante. L'intensité de la fluorescence de ces protéines n'est donc pas quantitative. Elles semblent donc difficilement utilisables aujourd'hui pour des analyses par FRET. Cependant, l'obtention future de telles protéines fluorescentes monomériques n'est pas à exclure. De telles protéines sont particulièrement adaptées à l'analyse de FRET avec un accepteur lipidique. En effet, avec un rendement quantique $\phi_{dsFP583}$ de 70% et un spectre d'émission de fluorescence compris entre 540 nm et 700 nm, ces protéines devraient fournir des valeurs élevées de $R_0$. Quelques candidats potentiels de FRET avec la dsFP583 sont présentés dans le Tableau 10.

TABLEAU 10

| Accepteur | Source / Référence | Rayon de Förster Donneur / Accepteur $R_0$, en Å | $\lambda_{exc}{}^{Donneur}$ $\epsilon$ Accepteur à $\lambda_{exc}$ du Donneur | Propriétés |
|---|---|---|---|---|
| Texas Red® DHPE N-(Texas Red®)-1,2-dihexadécanoyl-phosphatidyléthanolamine | Molecular Probes Leiden, Pays-Bas T-1395 l'accepteur | Nd | | Lipide membranaire Rendement quantique de $\phi_f{}^{donneur} = 0,29$ |
| Texas Red®-X, SE Ester Succinimidylique du Texas Red®-X | Molecular Probes Leiden, Pays-Bas T-6134 | Nd | | Fluorophore greffable sur Lipide ou protéine membranaires Rendement quantique de l'accepteur, $\phi_f{}^{donneur} = 0,29$ |

3 - Lipide fluorescent (Donneur) / EGFP (Accepteur) dans le cas de FRET

Compte tenu des propriétés de fluorescence de la EGFP (Clontech Laboratories Inc., Palo Alto CA (USA)), avec un maximum d'absorption à 475 nm, une émission de fluorescence à 507 nm, un rendement quantique de 60% et un coefficient d'extinction moléculaire de 55000 $M^{-1}.cm^{-1}$ à 475 nm, il apparaît intéressant de considérer la EGFP comme accepteur de FRET. Le Tableau 11 présente une sélection de lipides fluorescents qui ont été choisis pour la qualité du recouvrement spectral de leur émission de fluorescence avec l'absorption de la EGFP. La sonde DPH est un candidat Donneur très performant car son rendement quantique de fluorescence de 90% est particulièrement élevé. En parallèle, le rendement quantique élevé de l'Accepteur (EGFP) permet une bonne détection de l'apparition de la fluorescence de l'Accepteur dans l'expérience de FRET. Cette propriété représente un avantage important pour la caractérisation du mécanisme intime du FRET en permettant la mesure simultanée de la fluorescence du Donneur et de la fluorescence de l'Accepteur.

Les sondes dérivées de l'acétyle-2-anthracène qui présentent des bandes intenses d'absorption à 360 nm, 380 nm et 400 nm et une émission de fluorescence dans les membranes à 460 nm peuvent être avantageusement intégrées dans les lipides membranaires par les voies métaboliques de la cellule vivante.

TABLEAU 11

| Donneur | Source / Référence | Rayon de Förster Don / Acc $R_0$, en Å | $\lambda_{exc}{}^{Donneur}$ $\epsilon$ Accepteur à $\lambda_{exc}$ du Donneur | Propriétés |
|---|---|---|---|---|
| DPH | Molecular Probes | | .360nm | Sonde membranaire |

(suite)

| Donneur | Source / Référence | Rayon de Förster Don / Acc $R_0$, en Å | $\lambda_{exc}^{Donneur}$ ε Accepteur à $\lambda_{exc}$ du Donneur | Propriétés |
|---------|-------------------|------------------------------------|-----------------------------------------------------------|------------|
| diphényl-1,6-hexatriène-1,3,5 | Leiden, Pays-Bas D-202 | 49,2 | 1320 $M^{-1}.cm^{-1}$ | Non fluorescente dans l'eau $\phi_f^{acc} = 0,80$ |
| Prodan Propionyle-6-méthyl amino-2-naptalène | Molecular Probes Leiden, Pays-Bas P-248 | nd | 360nm 1320 $M^{-1}.cm^{-1}$ | Sonde membranaire |
| Laurdan Dodecanoyl-6-diméthyl amino-2-naptalène | Molecular Probes Leiden, Pays-Bas D-250 | nd | 360nm 1320 $M^{-1}.cm^{-1}$ | Sonde membranaire |

4 - Luciférase (Donneur) / Lipide fluorescent (Accepteur) dans le cas de BRET

Deux candidats donneurs de luminescence sont les luciférases de *Renilla reniformis* (BioSignal Packard, Montreal, Canada) et celle de *Oplophorus gracilirostris* (Shimomura et al (2000), FEBS Letters, 481, 19-25), dont l'émission de lumière est induite, non par une excitation lumineuse, mais par la réaction chimique d'un substrat (coelentérazine) capable de traverser les membranes de cellules vivantes. L'intérêt majeur de ces luciférases est leur totale indépendance vis à vis d'une lumière excitatrice et vis à vis d'autres cofacteurs, autres que le substrat. En absence de substrat, la luciférase n'émet pas de photons et l'accepteur n'est pas excité. En revanche, en présence de substrat coelentérazine, la luciférase transfert son énergie à l'accepteur avec apparition de signal de fluorescence caractéristique du BRET. L'avantage du BRET réside dans son principe même, à savoir une génération *ex nihilo* de fluorescence qui sera aisément détectée, même à bas niveau, en l'absence de lumière excitatrice parasite.

L'addition de substrat coelentérazine conduit à une émission lumineuse dans la zone de 460 à 490 nm dont le rendement quantique, défini par le nombre de photons émis par mole de substrat consommé, est de $\phi_{CL}$ (Rluc) = 5,5% pour la luciférase de *Renilla reniformis* et de $\phi_{CL}$ (Aluc) = 34% pour la luciférase de *Aplophorus gracilirostris*. Des mesures de BRET pourraient alors être possibles avec un Accepteur dont les longueurs d'onde d'absorption se situeraient autour de 480 nm.

Partenaires de la Luciférase

Trois lipides fluorescents ont été sélectionnés sur la base de la position de leurs spectres d'absorption et présentés dans le Tableau 12. Il s'agit d'une part d'un phospholipide dérivé du NBD (NBD-PE) et d'autre part des dérivés de sels de pyridinium hydrophobes. Les valeurs du $R_0$ sont plus élevées avec la luciférase de *Oplophorus gracilirostris* en raison de son rendement quantique plus élevé. Un candidat accepteur, le DiA, présente, avec la luciférase de *Oplophorus gracilirostris* une valeur de $R_0$ de 44,7 Å qui permet d'envisager une application de BRET. Le DiA est un cation lipophile, qui se localise sur le feuillet interne des membranes plasmiques sous l'effet du potentiel membranaire. La fluorescence du DiA émise entre 500 nm et 550 nm sera d'autant plus facilement analysée du fait que la luciférase n'exige pas d'excitation lumineuse.

TABLEAU 12

| Donneur | Accepteur | Source / Référence | Rayon de Förster Donneur/ Accepteur $R_0$, en Å | Propriétés |
|---------|-----------|-------------------|---------------------------------------------|------------|
| Luciférase de *Renilla reniformis* | di-8-ANEPPS | Molecular Probes Leiden, Pays-Bas D-3167 | 29,9 | sel interne |
| Luciférase de *Aplophorus gracilirostris* | di-8-ANEPPS | Molecular Probes Leiden, Pays-Bas D-3167 | 41,2 | sel interne |

(suite)

| Donneur | Accepteur | Source / Référence | Rayon de Förster Donneur/ Accepteur $R_0$, en Å | Propriétés |
|---|---|---|---|---|
| Luciférase de *Renilla Reniformis* | DiA | Molecular Probes Leiden, Pays-Bas D-3883 | 32,5 | Cation Lipophile |
| Luciférase de *Aplophorus Gracilirostris* | DiA | Molecular Probes Leiden, Pays-Bas D-3883 | 44,7 | Cation Lipophile |
| Luciférase de *Renilla Reniformis* | NBD-PE | Molecular Probes Leiden, Pays-Bas N-360 | 28,9 | Phospholipide membranaire |
| Luciférase de *Aplophorus Gracilirostris* | NBD-PE | Molecular Probes Leiden, Pays-Bas N-360 | 38,9 | phospholipide membranaire |

<u>5 - Utilisation de cryptates de métaux de transitions comme Donneurs du couple de FRET</u>

Récemment, ont été développés des complexes métalliques avec des Terres Rares (Europium, Terbium) présentant des propriétés de fluorescence adaptées au mécanisme de FRET. Ces complexes, pris comme Donneur dans les couples de FRET, ont une longue durée de vie d'état excité de fluorescence. Cet avantage permet de discriminer, sur une base temporelle, entre la fluorescence du cryptate et l'autofluorescence des cellules d'une part et entre la fluorescence du cryptate et la fluorescence résiduelle de l'Accepteur. L'article cité en référence décrit un couple Donneur/ Accepteur (Cryptate d'Europium/Allophycocyanine) dont la valeur du $R_0$ atteint 95 Å. [Alpha-Bazin A, Bazin H, Préaudat M, Trinquet E and Mathis G, (2001), 21, 439-455. Rare Earth Cryptates and TRACE Technology as Tools for Probing Molecular Interactions in Biology. In New Trends in Fluorescence Spectroscopy - Applications to Chemical and Life Sciences. B. Valeur and JC Brochon Eds, Springer-Verlag Berlin, Heidelberg, New-York]. Dans le cadre de l'invention, le cryptate métallique greffé sur une protéine est le Donneur de fluorescence tandis que le rôle de l'Accepteur est tenu par un dérivé de la fluorescéine émettant de la fluorescence au-delà de 650 nm.

<u>Exemple n° 2 : Mise en évidence de FRET entre la Cycle3-GFP-Akt et des liposomes marqués au DiI - Recrutement de la Cycle3-GFP-Akt à la surface de liposome induit par l'interaction entre le domaine PH (Pleckstrin Homology) de la Cycle3-GFP-Akt et les $PIP_3$ des liposomes</u>

Le but de cet exemple est de démontrer, sur un système modèle, un mécanisme de FRET entre un Donneur protéique (Cycle3-GFP-Akt) et un Accepteur lipidique (DiI) inséré dans la membrane lipidique de liposomes. Il renvoie directement aux exemples des Tableaux 1, 2 et 8 (« Protéines à domaines PH », « Protéines sans domaine PH mais avec domaine FYVE » et « Protéines associées à des membranes intracellulaires autres que la membrane plasmique ». La protéine Akt est normalement soluble et son recrutement à la membrane plasmique est induit par des phospholipides acides présents dans la membrane, les phosphatidylinositol-3,4,5-triphosphate ($PIP_3$) selon un processus qui met en oeuvre des interactions spécifiques entre les têtes polaires des $PIP_3$ et le domaine PH, (pour domaine homologue de celui de la pleckstrine) de l'Akt.

Cet exemple permet de montrer que le FRET (variation de la fluorescence du Donneur) observé est dû spécifiquement à la présence de l'Accepteur lipidique (DiI) inséré dans la membrane, mais aussi au rapprochement dans l'espace du DiI et de la Cycle3-GFP-Akt induit par la présence des molécules de $PIP_3$ à dans les liposomes. Il représente la base expérimentale fondamentale à la mise en oeuvre du procédé d'analyse du recrutement ou de la dissociation d'une protéine par rapport à la membrane plasmique de cellules vivantes. Par extension, cet exemple démontre les potentialités du procédé pour suivre le recrutement de protéines marquées sur d'autres systèmes membranaires que la membrane plasmique, à savoir, l'enveloppe nucléaire, le réticulum endoplasmique, le Golgi, les mitochondries (Tableau 8), les chloroplastes ou toutes vésicules des cellules eucaryotes ou encore la membrane plasmique des procaryotes.

<u>Construction du plasmide codant pour la protéine de fusion Cycle3-GFP-Akt</u>

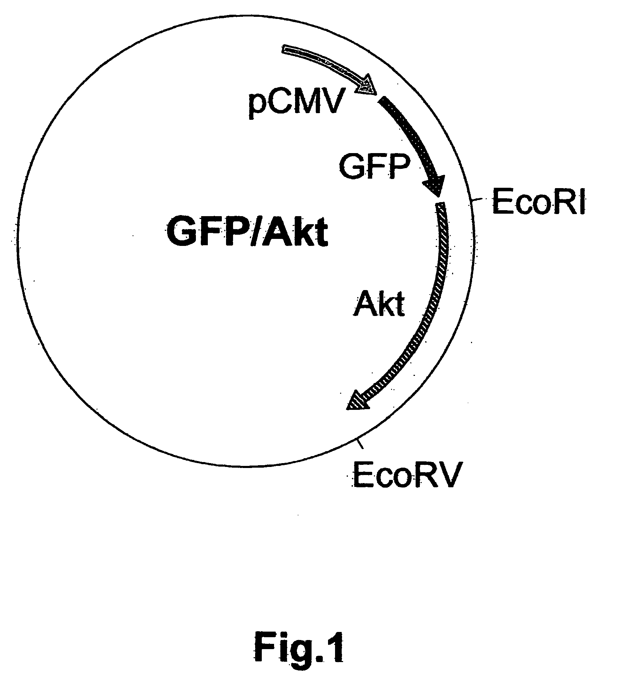

L'acide désoxyribonucléique complémentaire (ADNc) codant pour la protéine Akt/PKB$\alpha$ est obtenu après avoir effectué une transcription inverse couplée à une réaction de polymérisation en chaîne (RT-PCR) à partir d'acides ribonucléiques (ARN) extraits des cellules MCF-7. Les cellules MCF-7 sont ensemencées dans des boites de culture de 60 cm$^2$ à 1000000 de cellules par boite. Après 24 heure, les cellules sont lysées et les ARN extraits en utilisant le kit RNeasy mini kit (Qiagen, Courtaboeuf, France réf. 74104). On prépare 1 µg d'ARN dilué dans de l'eau distillée, dépourvue de DNases et de RNases, à une concentration finale de 0,2 µg/ml, la préparation est placée 10 minutes à 68°C puis transférée à 4°C (glace pillée). A partir de ces ARN, on synthétise des ADNc simples brins en réalisant une transcription inverse avec une amorce aléatoire. Pour cela, on ajoute aux ARN dilués de la RNasine® (40 ui/µl ; Promega, Charbonnières, France), du dithiothréitol à une concentration finale de 10 mM, un mélange de nucléotides à une concentration finale de 0,5 mM, l'amorce aléatoire (Invitrogen Life technologies, Cergy Pontoise, France Réf. 48190-011) à une concentration finale de 5 µM et l'enzyme Transcriptase Inverse RT-M-MLV (Moloney Murine Leukemia virus, (Invitrogen Life technologies, Cergy Pontoise, France réf. 28025-013). On incube ce mélange réactionnel pendant 1 heure à 37°C. En utilisant deux amorces appropriées, on amplifie la séquence codante de la protéine Akt par la technique de PCR. On mélange les ADNc simples brins avec une solution de nucléotides à une concentration finale de 200 µM, les deux amorces à une concentration finale de 0,5 µM et l'ADN polymérase qui est la DyNAzyme EXT® (New England Biolabs, Beverly MA, USA, réf: F505S) à une concentration finale de 0,02 UI/µl, le tout est placé dans un tampon optimisé dilué dans de l'eau. Après une dénaturation initiale réalisée pendant 10 minutes à 94°C, les ADNc doubles brins sont amplifiés par une phase de dénaturation d'une minute à 94°C puis une phase d'hybridation d'une minute à 60°C et une phase d'élongation d'une minute à 72°C, cette opération est renouvelée 30 fois. A la fin des 30 cycles, on effectue une élongation terminale de 10 minutes à 72°C. Le produit PCR de 1515 paires de bases, correspondant à la séquence codante de la protéine Akt, est ligaturé dans le vecteur pcDNA3.1/NT-GFP-TOPO (Invitrogen Life Technologies, Cergy Pontoise, France réf. K4810-01). On obtient alors une construction contenant un promoteur pCMV qui permet l'expression de la protéine de fusion Cycle3-GFP-Akt où la protéine Akt est clonée en aval de la protéine Cycle3-GFP. La carte du plasmide codant pour la Cycle3-GFP-Akt est représentée sur la Figure 1. Cette construction a été vérifiée par analyse enzymatique et validée par une transfection transitoire dans des cellules Cos-7.

Préparation de la Cycle3-GFP-Akt

Des cellules Cos-7 (ECACC n°87021302), cultivées au 4$^{ème}$ passage, sont ensemencées stérilement à 600 000 cellules par boite de culture plastique de 20 cm$^2$ (Nunclon®Delta). Le milieu de culture (5 ml par boite) est du DMEM dépourvu de rouge phénol (Invitrogen Life Technologies, Cergy Pontoise, France réf: 31053-028) et complémenté avec 10% (v/v) de sérum de veau foetal (Sigma-Aldrich Chimie Sarm, Saint-Quentin Fallavier, France réf: F-7524), de la L-glutamine à une concentration de 2 mM, de la Pénicilline à une concentration de 100 UI/ml et de la Streptomycine à une concentration de 100 µg/ml. 24 heures après l'ensemencement, les cellules sont transfectées par le plasmide Cycle3-GFP-Akt en utilisant la LipofectAMINE®2000 (Invitrogen Life Technolgies, Cergy Pontoise, France réf: 11668-019). Pour ce faire, on prépare 8 µg d'ADN plasmidique Cycle3-GFP-Akt dans 500 µl de milieu DMEM dépourvu de rouge phénol et complémenté par de la L-glutamine à une concentration de 2 mM. On homogénéise par vortex et on incube ce mélange pendant 5 minutes à 20°C. Par ailleurs, on prépare 20 µl de LipofectAMINE®2000 dans 500 µl de milieu DMEM dépourvu de rouge phénol et complémenté par de la L-glutamine à une concentration de 2 mM. On homogénéise par vortex et on incube ce mélange pendant 5 minutes à 20°C. On transfert la solution de LipofectAMINE®2000 dans la solution d'ADN et on incube ce mélange pendant 20 minutes à 20°C. Pendant ce temps, on lave les cellules avec 3 ml de DMEM dépourvu de rouge phénol et complémenté par de la L-glutamine à une concentration de 2 mM puis on leur ajoute 4 ml de DMEM dépourvu de rouge phénol et complémenté par de la L-glutamine à une concentration de 2 mM. Enfin, on ajoute le mélange de transfection (1 ml) sur les cellules, on homogénéise et on incube les cellules pendant 4 heures à 37°C sous 5% de CO$_2$. On élimine le milieu de transfection et le remplace par 5 ml de milieu DMEM sans rouge phénol, complémenté avec 10% de sérum de veau foetal, L-glutamine, pénicilline et streptomycine. Les cellules sont replacées dans l'incubateur à 37°C sous 5% de CO$_2$ pendant 24 heures. On élimine le milieu de culture des cellules Cos-7 par aspiration et on lave les cellules deux fois avec 1 ml de Tampon Phosphate Salin (PBS) et on reprend les cellules avec 0,5 ml de tampon HEPES 25 mM, EDTA 20 mM, pH 7,4 contenant de l'orthovanadate 1 mM, Aprotinine 10 µg, PMSF 0,1 mM et Leupeptine 10 µg. On gratte les cellules avec un racloir en plastique et on reprend la suspension de cellules dans un tube Eppendorf. Cette suspension est agitée vigoureusement au vortex pendant 5 secondes puis centrifugée 5 minutes à 4°C à une vitesse de 13000 tours par minute. Le surnageant résultant est analysé par spectrofluorimétrie (SPF-500C® ; Bioritech ; France). Le spectre d'émission de fluorescence est enregistré entre 420 nm et 700 nm (bande passante de 5 nm) sous excitation à 398 nm (bande passante de 7,5 nm). Le spectre d'excitation de fluorescence est obtenu par observation de la fluorescence émise à 510 nm (bande passante de 5 nm) pour une lumière d'excitation variant entre 300 nm et 495 nm (bande passante de 7,5 nm). Les spectres d'excitation et d'émission de fluorescence sont caractéristiques de la fluorescence de la Cycle3-GFP en solution.

Réparation de liposomes EPC/Chol/PS/SM/PE/DiI/PIP$_3$ ; 40/20/10/10/20/2,5/5 ; mole/mole

On prépare des liposomes à partir d'un mélange de 4,4 mg de phosphatidylcholine de jaune d'oeuf (Fluka BioChemica, Saint Quentin Fallavier, France, réf: 61753), 2,4 mg de phosphatidyléthanolamine (Fluka BioChemica, Saint Quentin Fallavier, France, réf: 60647), 1,3 mg de cholestérol (Fluka BioChemica Saint Quentin Fallavier, France, réf: 26732), 1,2 mg de DL-$\alpha$-dipalmitoyl-phosphatidyl-L-sérine (Sigma-Aldrich Chimie Sarl, Saint Quentin Fallavier, France, Réf: P-1902), 1,1 mg de sphingomyéline (Sigma-Aldrich Chimie Sarl, Saint Quentin Fallavier, France, Réf: S-7004) et 57 mg d'HECAMEG® (Vegatec, Villejuif, France) que l'on solubilise dans 1,8 ml de chloroforme et 0,2 ml de méthanol. Les lipides sont séchés dans un ballon sous évaporateur rotatif à 40°C puis repris dans 5 ml d'eau. A partir de cette solution mère, on prépare 3 tubes à hémolyse en verre contenant chacun 500 $\mu$g de lipides (250 $\mu$l). On prépare aussi 2 mg de DiI (dioctadécyl-1,1'-tétraméthyl-3,3,3',3'-indocarbocyanine, Molecular Probes, Leiden, Pays-Bas, Réf: D-282) solubilisé dans 1 ml de diméthylformamide et 100 $\mu$g de PIP$_3$ (L-$\alpha$-dipalmitoyl-phosphatidylinositol-3,4,5-triphosphate, Calbiochem, Darmstadt, Allemagne, Réf: 524615) solubilisés dans 200 $\mu$l de diméthylsulfoxyde (DMSO).

|  | Lipides | DiI (2$\mu$g/$\mu$l) | DMF | PIP$_3$ | DMSO |
|---|---|---|---|---|---|
| Essai 1 | 500 $\mu$g | 10 $\mu$l | 0 $\mu$l | 100 $\mu$l | 0 $\mu$l |
| Essai 2 | 500 $\mu$g | 10 $\mu$l | 0 $\mu$l | 0 $\mu$l | 100 $\mu$l |
| Essai 3 | 500 $\mu$g | 0 $\mu$l | 10 $\mu$l | 100 $\mu$l | 0 $\mu$l |

Le pourcentage molaire, exprimé en mole de PIP$_3$ par mole de lipides totaux est de 5% dans les essais 1 et 3 et de 0% dans l'essai 2. Le pourcentage molaire, exprimé en mole de DiI par mole de lipides totaux est de 2,5% dans les essais 1 et 2. On homogénéise les trois mélanges et on place les solutions dans des boudins de dialyse (Spectra/Por®CE de seuil de coupure à 2000Da). On les place dans 1 litre d'eau distillée en présence de 3 grammes d'absorbant BioBeads®SM-2 (Bio-Rad Laboratoires, Marnes la Coquette, France) sous agitation magnétique à 4°C pendant 4 heures. On récupère ainsi les essais 1, 2 et 3 dépourvus de détergent et contenant des liposomes.

Mise en évidence du FRET

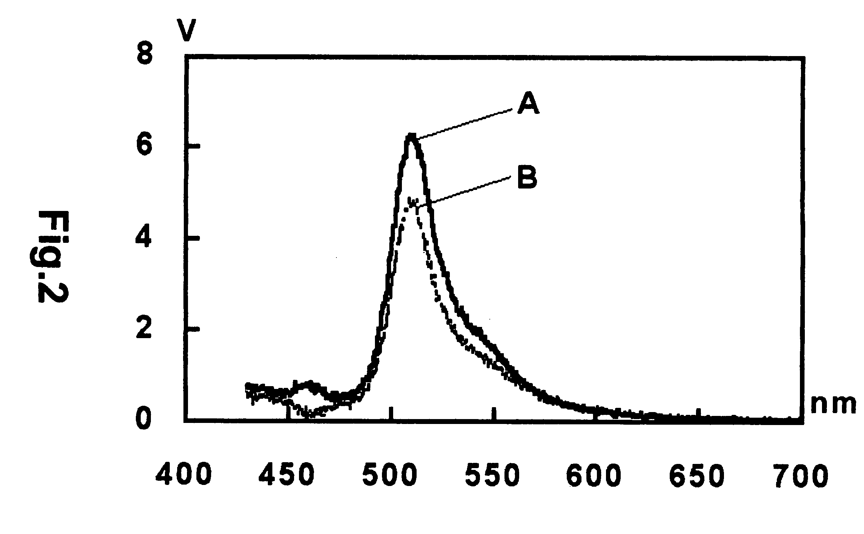

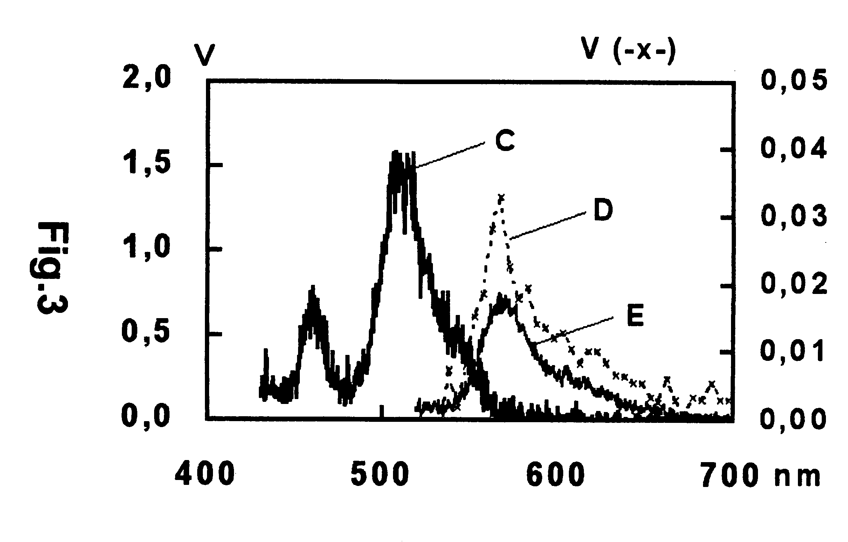

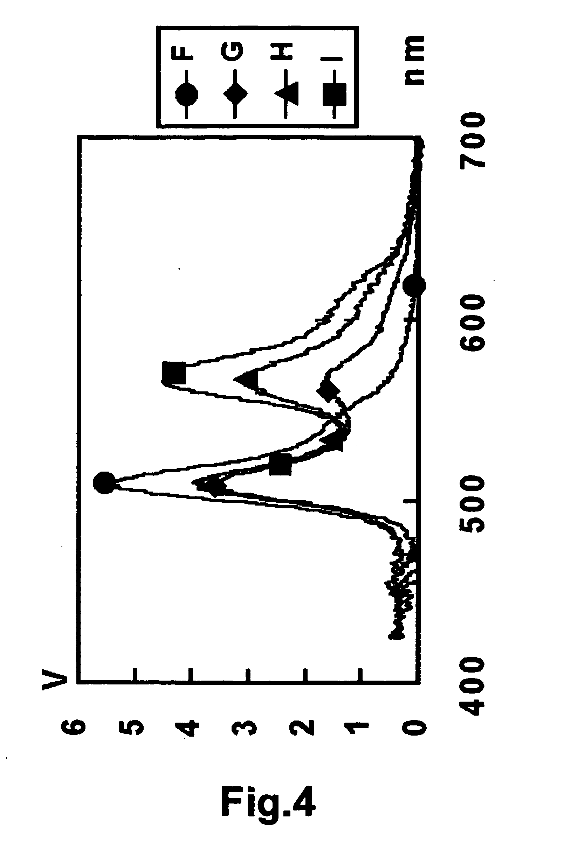

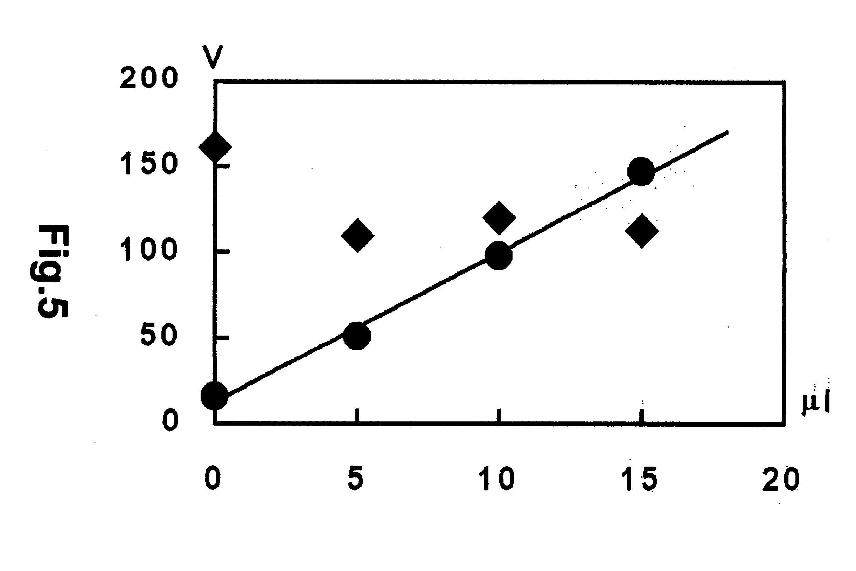

Dans une cuve adaptée à la spectrométrie de fluorescence on dépose 300 $\mu$l de tampon PBS et 60 $\mu$l de la solution de Cycle3-GFP-Akt. On enregistre le spectre d'émission de fluorescence à l'aide d'un spectrofluorimètre SPF-500C® entre 430 nm et 700 nm (bande passante de 7,5 nm). La longueur d'onde d'excitation est de 398 nm (bande passante de 7,5 nm). On retranche à ce spectre la contribution à la fluorescence du tampon PBS enregistré dans les mêmes conditions. Le spectre résultant, représenté en A sur la Figure 2, donne la valeur de la fluorescence exprimée en volts en fonction de la longueur d'onde exprimée en nm. On ajoute ensuite 5 $\mu$l de l'essai 1, on homogénéise le mélange et on enregistre le spectre de fluorescence dans les mêmes conditions que précédemment. On retranche la contribution du PBS à là fluorescence ainsi que la contribution à la fluorescence de 5 $\mu$l de liposomes de l'essai 1 mélangés avec 360 $\mu$l de tampon. Le spectre résultant est représenté en B (Figure 2). On observe une diminution de 25% de la fluorescence de la Cycle3-GFP-Akt en présence de 5 $\mu$l (10 $\mu$g) de liposomes contenant le DiI et le PIP$_3$.
Cependant, l'observation directe des spectres A et B (Figure 2) ne permet pas de confirmer la réalité du FRET. En effet, en raison de la valeur faible du rendement quantique de fluorescence de l'accepteur (5% < $\phi_{DiI}$ < 15%) il n'est pas possible de détecter directement l'apparition de la fluorescence de l'accepteur (DiI). Pour une analyse plus fine, on représente en C (Figure 3) le spectre de différence A - B sur lequel on observe le signal caractéristique de la Cycle3-GFP-Akt. On normalise ensuite les spectres A et B à la longueur d'onde de 510 nm et on calcule le spectre de différence B - A. Ce spectre est représenté en D (Figure 3) et est caractéristique de la fluorescence du DiI dont un spectre est représenté en E (Figure 3). On mesure l'intensité de la fluorescence apparaissant à 570 nm (0,04 volts) et l'intensité de fluorescence disparaissant à 510 nm (1,5 volts). On observe que la disparition de la fluorescence de la Cycle3-GFP-Akt est 37 fois supérieure à l'apparition de la fluorescence du DiI, ce qui compte tenu du rendement quantique de la Cycle3-GFP ($\phi_{Cycle3-GFP}$ = 70%), laisse présager un rendement quantique du DiI de 2% à 3%, proche des données de la littérature.
Etant donnée la faiblesse de l'intensité de la fluorescence du DiI apparue sur le spectre D (Figure 3), il est possible que la diminution de la fluorescence de la Cycle3-GFP-Akt soit due à du transfert radiatif de fluorescence dû au DiI. Les photons émis par la Cycle3-GFP-Akt seraient alors partiellement absorbés par le DiI conduisant à la diminution de la fluorescence de la Cycle3-GFP-Akt sans qu'un rapprochement dans l'espace ne soit nécessaire entre la Cycle3-GFP-Akt et le DiI. Un tel phénomène de filtre interne dépendrait directement de la concentration en DiI. Afin d'exclure cette possibilité, on observe l'effet de l'addition successive de 5 $\mu$l, 10 $\mu$l et 15 $\mu$l de liposomes de l'essai 1 sur la fluorescence de la Cycle3-GFP-Akt obtenue par excitation à 398 nm. Sur la Figure 4, les spectres F (0 µl), G (5 µl), H (10 µl) et I (15 µl) montrent une diminution de 30% de la fluorescence de la Cycle3-GFP-Akt en présence de 5µl de l'essai 1, tandis que la fluorescence de la Cycle3-GFP-Akt reste constante pour des volumes supérieurs d'essai. Sur la Figure 5, sont représentées les valeurs, en volts, d'intensité de la fluorescence à 510 nm (losanges) et à 570 nm (disques pleins). On observe ainsi l'augmentation proportionnelle au volume d'essai 1 de la fluorescence du DiI à 570 nm par excitation à 398 nm et la stabilisation du signal de la Cycle3-GFP-Akt à 510 nm. L'inhibition de la fluorescence de la Cycle3-GFP-Akt n'est donc pas proportionnelle à la concentration en DiI. Le phénomène observé est donc du FRET entre la Cycle3-GFP-Akt et le DiI.

Enfin, on montre que l'Essai 2 (liposomes sans PIP$_3$) et l'Essai 3 (liposomes sans DiI) sont sans influence sur la fluorescence de la Cycle3-GFP-Akt dans les mêmes conditions de concentration.

On conclut que l'extinction partielle de la fluorescence de la Cycle3-GFP-Akt et l'apparition de la fluorescence du DiI sont dues à un transfert non radiatif de fluorescence (FRET) spécifiquement entre la Cycle3-GFP-Akt et le DiI et que ce transfert est induit par le recrutement de la Cycle3-GFP-Akt à la surface des liposomes contenant du DiI.

Exemple n° 3 : Mise en évidence du FRET entre le récepteur membranaire MOR-1 (« Mu Opioid Receptor-1 ») couplé à la Cycle3-GFP, exprimé dans des cellules Cos-7 en culture, et la sonde membranaire DiI

Cet exemple permet de mettre en évidence, dans des cellules vivantes, l'existence d'un mécanisme de FRET entre un récepteur constitutivement membranaire de la famille des récepteurs couplés aux protéines G et couplé à la Cycle3-GFP et des lipides membranaires marqués avec la sonde fluorescente DiI. Le récepteur MOR-1 est une protéine constituée de 7 hélices α hydrophobes qui lui assurent une localisation exclusivement transmembranaire. Cet exemple permet aussi d'appuyer les applications possibles données dans le Tableau 7, concernant des protéines intégrales de la membrane contenant des peptides susceptibles d'être libérés par protéolyse dans le milieu extra ou intracellulaire. On peut envisager de coupler la GFP au fragment libérable de la protéine intégrale. Dans ce cas, à partir d'un état initial dans lequel les deux partenaires fluorescents sont proches et présentent un FRET important, le clivage d'un fragment de la protéine membranaire conduit à un état final dans lequel le partenaire peptidique libéré dans le cytoplasme ou dans l'espace péricellulaire s'éloigne du partenaire lipidique membranaire et entraîne la disparition du FRET.

Construction du plasmide (MOR-1-Cycle3-GFP) codant pour une protéine de fusion MOR-1-Cycle3-GFP

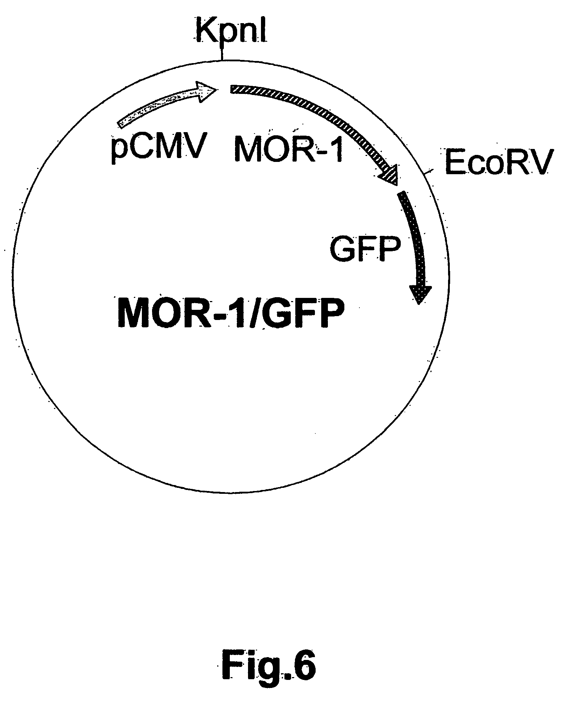

L'ADNc codant pour la protéine MOR-1 a été obtenu après PCR à partir du plasmide pcDNAI/MOR-I (don gracieux de Meunier JC, IPBS, Toulouse). Le produit PCR de 1212 paires de bases, correspondant à la séquence codante de la protéine MOR-1, est ligaturé dans le vecteur pcDNA/CT-GFP-TOPO (Invitrogen K4820-01). La construction contient le gène de résistance à l'ampicilline, un promoteur pCMV (cytomegalovirus) qui permet l'expression de la protéine de fusion MOR-1-Cycle3-GFP où la protéine MOR-1 est clonée en amont de la protéine Cycle3-GFP. La carte du plasmide codant pour la MOR-1-Cycle3-GFP est représentée sur la Figure 6. Cette construction a été vérifiée par analyse enzymatique et validée par transfection transitoire dans des cellules Cos-7.

Mise en oeuvre des cellules Cos-7 et transfection par l'ADN (MOR-1-Cycle3-GFP)

On ensemence une boite de culture plastique de 20 cm$^2$ de surface (Nunclon®Delta), qui a été préalablement garnie avec une lamelle en verre de taille 35 mm x 35 mm, avec 600000 cellules Cos-7 (ECACC n°87021302), cultivées au 4$^{ème}$ passage, dans les mêmes conditions que celles décrites dans l'exemple 2. 24 heures après l'ensemencement, les cellules sont transfectées par le plasmide MOR-1-Cycle3-GFP en utilisant la LipofectAMINE®2000 (Invitrogen Life Technolgies, Cergy Pontoise, France Réf: 11668-019) dans les mêmes conditions que celles décrites dans l'exemple 2. Les cellules sont observées 24 heures après la fin de la transfection.

Observation des cellules transfectées sous microscope à fluorescence Zeiss AXIOPLAN - Localisation membranaire du MOR-1-Cycle3-GFP

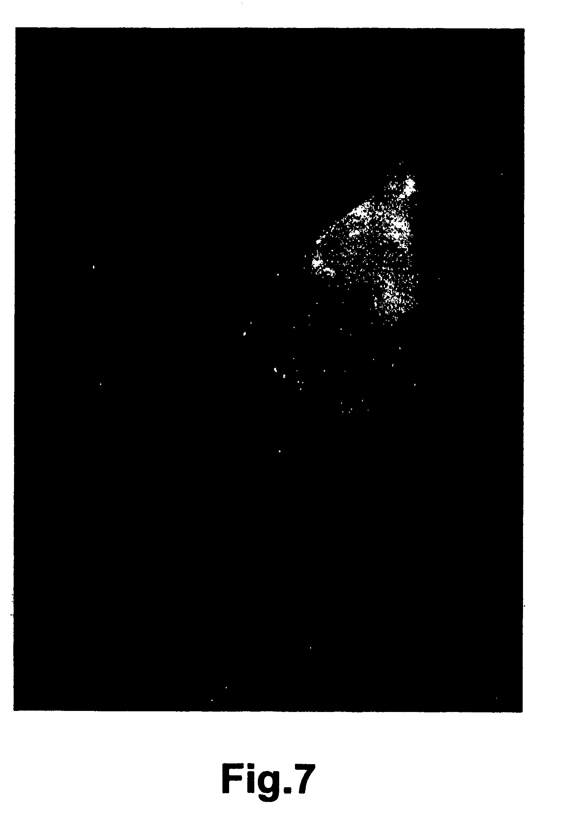

On élimine, par aspiration, le milieu de culture des cellules Cos-7 cultivées sur lamelle de verre 35 mm x 35 mm et on lave les cellules deux fois avec 1 ml de PBS. La lamelle supportant les cellules est placée dans une « chambre de Rose » (Rose G, (1954), Tex. Rep. Biol. Med., 12, 1074-1083. A separable and multipurpose culture chamber), remplie de milieu de culture DMEM dépourvu de rouge phénol mais complémenté avec 10% (v/v) de sérum de veau foetal (SVF), de la L-glutamine, de la Pénicilline et de la Streptomycine et permettant l'observation microscopique sur plusieurs heures de cellules vivantes en culture ainsi que le renouvellement du milieu de culture. L'observation des cellules est réalisée au moyen d'un microscope à fluorescence droit (Zeiss Axioplan) équipé d'un objectif à immersion Gx40 et d'une caméra CCD. La longueur d'onde d'excitation est de 400 nm (filtre d'excitation 405DF40, Omega Optical Inc, Brattleboro, USA), la fluorescence est observée à 510 nm (filtre d'émission 510DF60, Omega Optical Inc, Brattleboro, USA) et la lumière excitatrice est éliminée par réflexion en deçà de 455 nm (filtre dichroïque 455DRLP, Omega Optical Inc., Brattleboro, USA). On observe des cellules Cos-7 présentant une fluorescence de type membranaire intense en accord avec les données de la littérature (Figure 7).

Marquage des cellules dans la « Chambre de Rose » avec le DiI et caractérisation du marquage

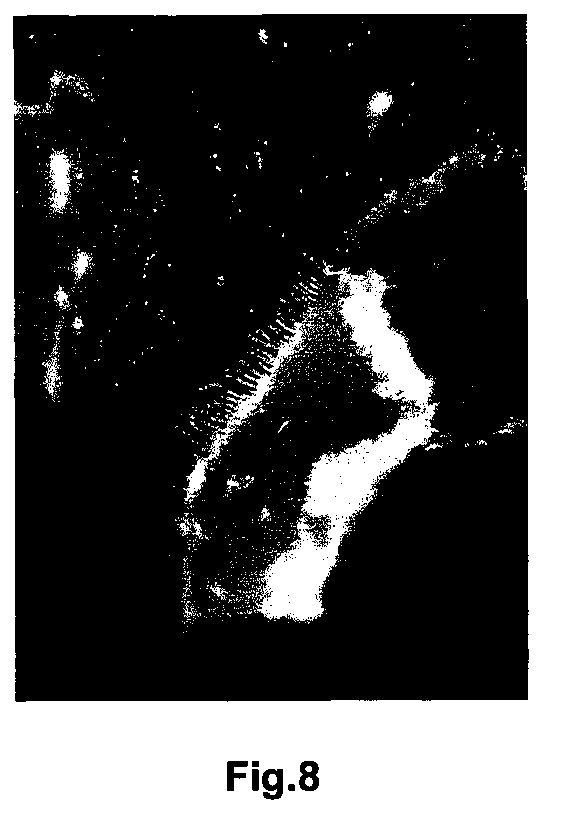

On démontre que les cellules Cos-7 transfectées avec le MOR-1-Cycle3-GFP peuvent être efficacement marquées par la sonde membranaire DiI sans affecter leur viabilité. Pour ce faire, les cellules Cos-7 observées précédemment sont conservées quelques minutes dans du milieu de culture. On prépare 5 ml de milieu DMEM dépourvu de rouge phénol et chauffé à 37°C. On ajoute 10 μl de SVF au milieu soit 0,2% (v/v). Le milieu DMEM/SVF 0,2% est maintenu à 37°C jusqu'à l'addition du marqueur membranaire fluorescent. On pèse 20 mg de DiI que l'on solubilise dans 2 ml de diméthylformamide. On prélève à l'aide d'une seringue de précision en verre 25 μl de la solution mère de DiI (250 μg de DiI) que l'on injecte lentement (25 μl/minute) et sous vortex dans le milieu DMEM/SVF 0,2% maintenu à 37°C. On obtient ainsi une solution limpide de DiI dans le milieu DMEM/SVF 0,2% que l'on refroidit à 20°C dans un bain thermostaté. Le milieu de marquage équilibré à 20°C contenant la DiI est injecté en 1 minute dans la chambre de Rose contenant les cellules et maintenue à 20°C au moyen d'une seringue de 10 ml. Le milieu de marquage est laissé au contact des cellules pendant 10 minutes à 20°C puis est éliminé par lavage avec 5 ml de DMEM dépourvu de rouge phénol, puis avec 10 ml de tampon phosphate 10 mM, NaCl 150 mM, pH 7,4. L'observation des cellules est réalisée au moyen d'un microscope à fluorescence droit (Zeiss Àxioplan) équipé d'un objectif à immersion Gx40 et d'une caméra CCD. La longueur d'onde d'excitation est de 546 nm, la fluorescence est observée au-delà de 590 nm et la lumière excitatrice est éliminée par réflexion en deçà de 580 nm. Le marquage fluorescent membranaire est observé sur la Figure 8.

Marquage, par la sonde fluorescente DiI, des membranes plasmiques de cellules Cos-7 en culture transfectées par l'ADN MOR-1-Cycle3-GFP et observation du FRET par spectroscopie de fluorescence

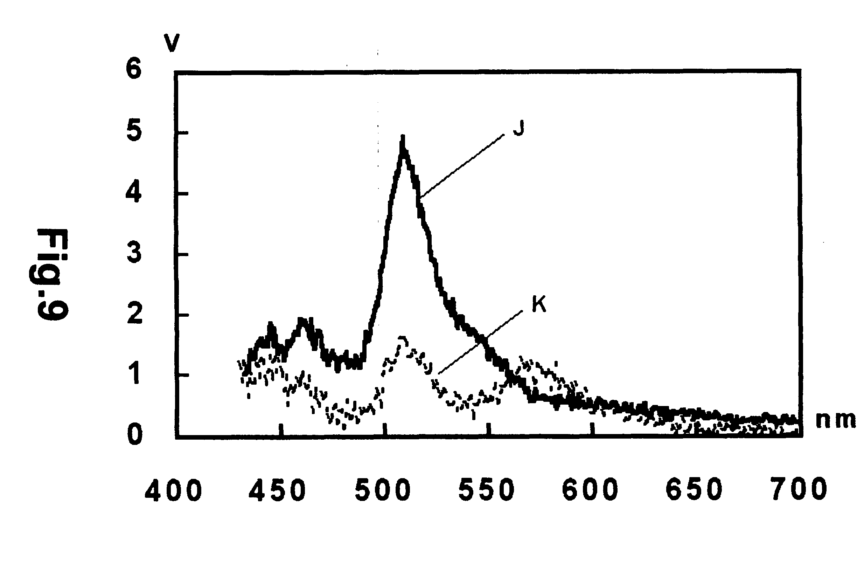

Les cellules Cos-7 cultivées sur plastique sont reprises 36 heures après la transfection et lavées deux fois avec 1 ml de PBS. Les cellules sont décrochées de la boite par addition de 1 ml d'une solution de trypsine / EDTA (Invitrogen Life Technologies, Cergy Pontoise, France Réf: 35400-019) diluée 10 fois dans du tampon Phosphate 10 mM, NaCl 150 mM, pH 7,4 et incubation pendant 10 minutes à 37°C. On ajoute 4 ml de milieu DMEM sans rouge phénol complémenté avec 10% de sérum de veau foetal et on finit de décoller les cellules par aspiration, refoulement successifs. On récupère la suspension cellulaire dans un tube à centrifuger de 15 ml. La suspension est centrifugée pendant 5 minutes à 20°C à une vitesse de rotation de 1500 tours par minute. Le surnageant est éliminé par aspiration et le culot est lavé 2 fois avec 2 ml de tampon PBS. Les cellules sont reprise dans 1 ml de tampon PBS et placées dans la cuve du spectrofluorimètre. La fluorescence des cellules est analysée entre 430 nm et 700 nm (bande passante 7,5 nm), sous excitation à 398 nm (bande passante 7,5 nm). On enregistre en parallèle et dans les mêmes conditions le spectre de fluorescence de cellules Cos-7 non transfectées et on calcule le spectre de différence représenté par la courbe J de la Figure 9. Les cellules transfectées avec MOR-1-Cycle3-GFP et les cellules non transfectées sont reprises dans la cuve de fluorescence, transvasées dans des tubes à centrifuger de 15 ml et centrifugées pendant 5 minutes à 20°C à une vitesse de rotation de 1500 tours par minute. On élimine le surnageant par aspiration et on ajoute 1 ml de milieu de marquage composé de 0,9 ml de milieu DMEM dépourvu de rouge phénol chauffé à 37°C, auquel on ajoute 100 μl d'une solution de BSA-FAF (Sérum Albumine Bovine Dépourvue d'Acides Gras, Sigma-Aldrich Chimie Sarl, Saint Quentin Fallavier, France, Réf: A-8806) à 1% dans du PBS. Le milieu DMEM/BSA est maintenu à 37°C jusqu'à l'addition du marqueur membranaire fluorescent. On pèse 20mg de DiI que l'on solubilise dans 2 ml de diméthylformamide. On prélève à l'aide d'une seringue de précision en verre, 5 μl de la solution mère de DiI (50 μg de DiI) que l'on injecte lentement (5 μl/minute) et sous vortex dans le milieu DMEM/BSA maintenu à 37°C. On obtient ainsi une solution limpide de DiI dans le milieu DMEM/BSA que l'on refroidi à 20°C dans un bain thermostaté. On incube les cellules dans le milieu de marquage pendant 10 minutes à 20°C sous agitation lente. On centrifuge les cellules pendant 5 minutes à 20°C à une vitesse de 1500 tours par minute, on élimine le surnageant par aspiration et on lave 2 fois les cellules avec 2 ml de PBS. Les cellules sont reprises dans 1 ml de PBS et analysées au spectrofluorimètre. La différence des spectres obtenus avec les cellules transfectées et les cellules non transfectées est représentée en K sur la Figure 9. On observe une forte diminution de la fluorescence de la MOR-1-Cycle3-GFP et une apparition simultanée d'un signal de fluorescence à 570 nm correspondant à l'apparition de FRET.

Cette caractérisation sans ambiguïté du FRET entre une protéine transmembranaire marquée avec la Cycle3-GFP et un marqueur membranaire démontre les potentialités du procédé pour détecter l'apparition ou la disparition d'interactions entre deux partenaires fluorescents, dont l'un est un lipide membranaire et l'autre une protéine.

Exemple n° 4 : Mise en évidence de FRET entre la protéine Raf-CAAX couplée à la Cycle3-GFP (Cycle3-GFP-Raf-CAAX), exprimée dans des cellules en culture Cos-7, et la sonde membranaire DiI.

Cet exemple a pour but d'étayer l'application du procédé au criblage de molécules présentant un effet sur la localisation de protéines cytosolubles mais interagissant avec la membrane plasmique par l'intermédiaire d'un ancrage lipidique dynamique. Il renvoie au Tableau 3 (Protéines sans domaine PH ou FYVE mais associées à la membrane plasmique par ancrage lipidique). La nature de cet ancrage peut être notamment une acylation (palmitoylation ou myristoylation) ou/et une isoprénylation (farnésylation ou géranylgéranylation).

La protéine Raf-1 est majoritairement localisée dans le cytoplasme de la cellule en absence de stimulation. Le motif CAAX est une séquence de 20 acides aminés de la protéine K-Ras. Ce motif CAAX est un site de modification post-traductionnelle qui est reconnu par l'enzyme conduisant à la farnésylation de la protéine et qui, en conjonction avec un site polybasique de 6 lysines, permet l'ancrage de K-Ras à la membrane. La protéine Cycle3-GFP-RafCAAX est constituée de la Cycle3-GFP fusionnée avec la protéine Raf-1 et avec le motif CAAX. La construction de la Cycle3-GFP-RafCAAX conduit à l'expression d'une protéine Cycle3-GFP-Raf-1 constitutivement localisée à la membrane plasmique des cellules, par l'intermédiaire d'un ancrage dynamique mettant en jeu un groupement isoprénoïde hydrophobe.

Construction du plasmide codant pour une protéine de fusion Cycle3-GFP-RafCAAX

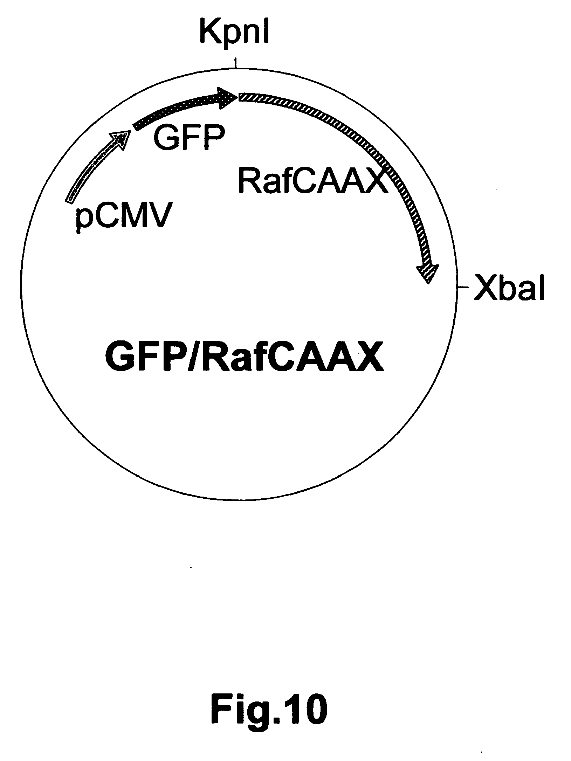

L'ADNc codant pour la protéine RafCAAX est commercialisé par la société Clontech (Clontech Laboratories Inc) K6015-1 sous le nom pCMV-RafCAAX. 3 $\mu$g de cet ADNc sont digérés de façon simultanée par les enzymes de restriction XhoI (New England Biolabs, Beverly, MA, USA), à raison de 3 UI/$\mu$g d'ADN et BamHI (New England Biolabs, Beverly, MA, USA) à raison de 3 UI/$\mu$g d'ADN, pendant 2 heures à 37°C, libérant un fragment de 2026 paires de bases qui correspond à la séquence codante de la protéine RafCAAX. Les extrémités 5' et 3' de ce fragment sont rendues bout franc par l'addition de la polymérase de Klenow (New England Biolabs, Beverly, MA, USA Réf : MS0210S), à raison de 1 UI/$\mu$g d'ADN pendant 30 minutes à 37°C en présence d'un mélange de nucléotides à une concentration finale de 33 $\mu$M. Par ailleurs, le vecteur pcDNA3.1/NT-GFP (Invitrogen Life Technologies, Cergy Pontoise, France, Réf: K4810-01) est digéré par les enzymes de restriction KpnI (New England Biolabs, Beverly, MA, USA) à raison de 3 UI/$\mu$g d'ADN et XbaI (New England Biolabs, Beverly, MA, USA) à raison de 3 UI/$\mu$g d'ADN libérant un oligonucléotide de 70 paires de bases qui est remplacé par un oligonucléotide contenant 40 paires de bases appelé $GFP_K$, permettant ultérieurement un clonage dans le même cadre de lecture des protéines GFP et RafCAAX. Ce vecteur $GFP_K$ est digéré par l'enzyme EcoRV (New England Biolabs, Beverly, MA, USA) à raison de 3 UI/$\mu$g d'ADN, déphosphorylé par la phosphatase alcaline (1UI/$\mu$g d'ADN) pendant 30 minutes à 37°C puis ligaturé en présence du fragment RafCAAX de 2026 paires de bases. Cette construction contient un promoteur pCMV (cytomégalovirus) qui permet l'expression de la protéine de fusion GFP-RafCAAX dans laquelle la protéine RafCAAX est clonée en aval de la protéine Cycle3-GFP. Cette construction a été vérifiée par analyse enzymatique et validée par transfection transitoire dans des cellules Cos-7. La carte du plasmide codant pour la protéine GFP-RafCAAX est représentée sur la Figure 10.

Mise en oeuvre des cellules Cos-7 et transfection par l'ADN GFP-RaPCAAX

Cette étape est similaire de celle réalisée pour la transfection de l'ADN MOR-1-Cycle3-GFP et est décrite dans l'exemple 3. Les cellules sont observées 24 heures après la fin de la transfection.

Visualisation de la localisation membranaire de la protéine GFP-RafCAAX

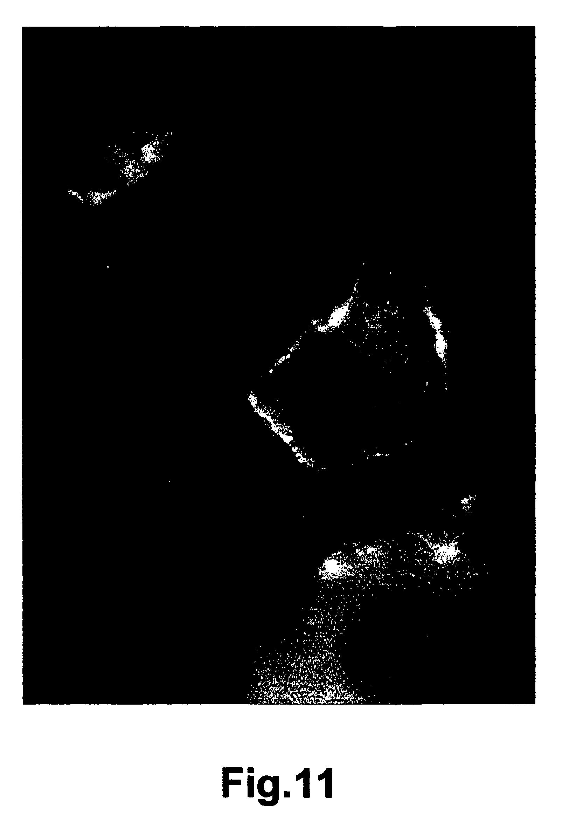

On élimine, par aspiration, le milieu de culture des cellules et on les lave deux fois avec 1 ml de Tampon Phosphate Salin. La lamelle supportant les cellules est placée dans une « chambre de Rose », remplie de milieu de culture DMEM dépourvu de rouge phénol mais complémenté avec 10% (v/v) de sérum de veau foetal, de la L-glutamine, de la Pénicilline et de la Streptomycine. L'observation des cellules est réalisée au moyen d'un microscope à fluorescence droit Axioplan (Carl Zeiss, LePecq, France) comme indiqué dans l'exemple 3. On observe des cellules Cos-7 présentant une fluorescence de type membranaire comme sur la Figure 11 alors que la fluorescence observée sur des cellules transfectées avec GFP-Raf, dépourvu de CAAX, reste exclusivement cytoplasmique.

Marquage *in vivo* des cellules Cos-7 transfectées (GFP-RafCAAX) avec le DiI

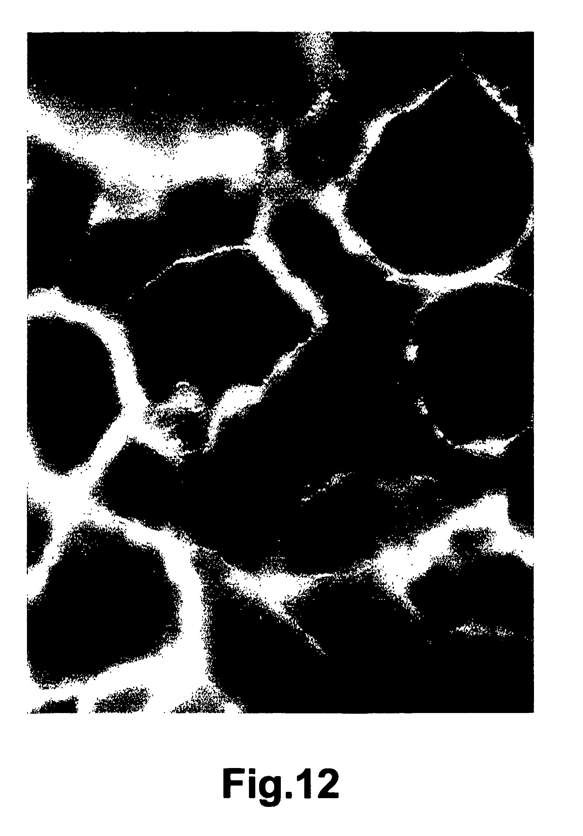

On prépare 5 ml de milieu DMEM/SVF 0,2%/DiI (250 $\mu$g) comme décrit dans l'exemple 3. On injecte les 5 ml de milieu de marquage au moyen d'une seringue de 10 ml en 1 minute. Le milieu de marquage est laissé au contact des cellules pendant 10 minutes à 20°C puis est éliminé par lavage avec 5 ml de DMEM dépourvu de rouge phénol, puis avec 10 ml de tampon phosphate 10 mM, NaCl 150 mM, pH 7,4. Le marquage fluorescent membranaire est observé sur la Figure 12.

Analyse quantitative du FRET entre la Cycle3-GFP-RafCAAX et la sonde membranaire DiI par spectrofluorimétrie

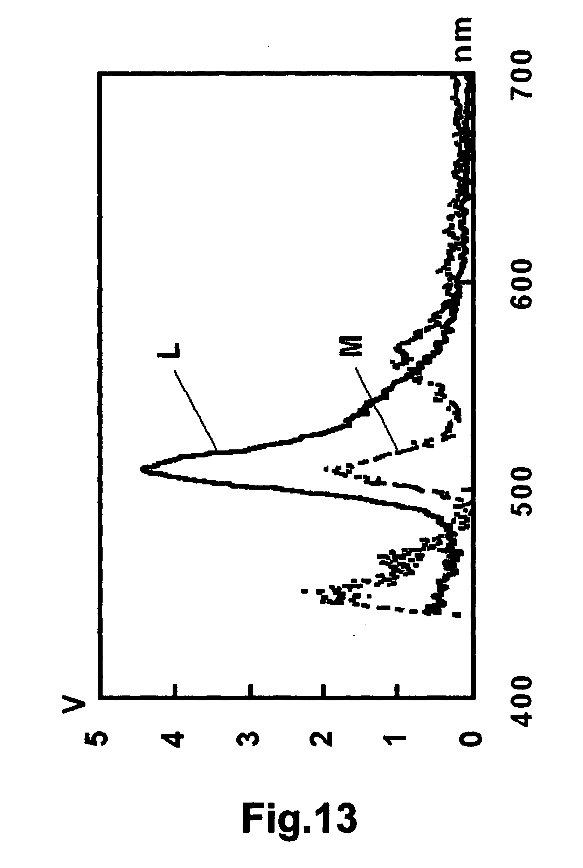

Des cellules Cos-7 cultivées dans deux boites de 20 cm$^2$ de surface, transfectées ou non avec le plasmide GFP-RafCAAX sont décrochées de leur support par traitement à la trypsine/EDTA, puis marquées avec le DiI de la même façon que dans l'exemple 3 et placées dans une cuve de spectrofluorescence. La fluorescence des cellules transfectées est analysée entre 430 nm et 700 nm (bande passante de 7,5 nm), sous excitation à 398 nm (bande passante de 7,5 nm). On enregistre en parallèle et dans les mêmes conditions le spectre de fluorescence de cellules Cos-7 non transfectées. On calcule le spectre de différence entre les cellules transfectées et les cellules non transfectées (Figure 13, spectre L). Les cellules transfectées avec la Cycle3-GFP-RafCAAX et les cellules non transfectées sont transvasées dans des tubes de 15 ml et centrifugées pendant 5 minutes à 20°C à une vitesse de rotation de 1500 tours par minute. On élimine le surnageant par aspiration et on ajoute 1 ml de milieu de marquage composé obtenu selon le même protocole que celui décrit dans l'exemple 3. On incube les cellules dans le milieu de marquage pendant 10 minutes à 20°C sous agitation lente. On centrifuge les cellules pendant 5 minutes à 20°C à une vitesse de 1500 tours par minute, on élimine le surnageant par aspiration et on lave les cellules 2 fois avec 2 ml de Tampon Phosphate Salin. Les cellules transfectées et les cellules non transfectées sont reprises dans 1 ml de PBS et analysées au spectrofluorimètre. On retranche au spectre de fluorescence des cellules transfectées, la contribution des cellules non transfectées (Figure 13, spectre M). On observe une forte diminution de la fluorescence de la Cycle3-GFP-RafCAAX en présence de DiI mais aussi l'apparition simultanée d'un signal caractéristique du DiI. La conjonction de la diminution de la fluorescence de la GFP avec l'apparition de la fluorescence du DiI est la preuve formelle de l'existence d'un FRET entre la protéine Cycle3-GFP-RafCAAX localisée à la membrane grâce à son motif farnésyle et la sonde lipidique DiI, elle aussi localisée à la membrane.

Exemple n° 5 : Analyse par FRET de la translocation de la Cycle3-GFP-PKCII vers la membrane plasmique induite par le traitement par un ester de phorbol de cellules vivantes transfectées transitoirement avec le gène de la Cycle3-GFP-PKCII et dont la membrane est marquée avec la sonde membranaire DiI

Cet exemple a pour but d'étayer l'application du procédé de criblage de molécules présentant un effet sur la relocalisation de protéines de transduction cytosoliques vers la membrane plasmique. Il renvoie aux tableaux 1 (protéines à domaines PH), 2 (protéines sans domaine PH mais à domaine FYVE, C1 ou C2), 3 (protéines sans domaine PH ou FYVE ou C1 ou C2 mais associées à la membrane plasmique par ancrage lipidique), 4 (protéines sans domaines PH ou FYVE ou C1 ou C2, sans ancrage membranaire mais associées directement à des récepteurs membranaires), 5 (protéines sans domaines PH ou FYVE ou C1 ou C2, sans ancrage membranaire mais associées indirectement à des récepteurs membranaires) et 6 (protéines associées à la membrane plasmique mais selon un mécanisme différent de ceux décrits dans les tableaux 1 à 5).

La famille des protéines kinase C englobe des protéines dont l'activité conduit à la phosphorylation, à partir d'ATP, de résidus sérine et/ou thréonine sur des protéines cellulaires. Cette famille se compose d'une dizaine d'isoformes se répartissant en plusieurs sous types : les PKC dites « conventionnelles » ($\alpha$, $\beta$I, $\beta$II, et y) qui sont régulées et activées par le calcium et le diacylglycérol, les PKC dites « nouvelles » ($\delta$, $\varepsilon$, $\eta$, $\theta$) qui sont activées par le diacylglycérol uniquement et les PKC dites « atypiques » ($\zeta$ et $\iota$/$\lambda$) dont l'activation est indépendante du calcium et du diacylglycérol. Les différentes isoformes de la PKC diffèrent entre elles par leur structure, leur distribution tissulaire, leur localisation intracellulaire et leur spécificité de substrats. Les substrats des PKC sont nombreux et interviennent dans des processus biologiques aussi variés que la prolifération cellulaire, la différenciation et l'apoptose.

La PKC$\beta$II est une protéine cytosolique qui est recrutée à la membrane plasmique au cours du processus d'activation de la voie de transduction liée à la phospholipase C (PLC$\beta$) sous la dépendance d'un récepteur couplé aux protéines G (RCPG). Schématiquement, la liaison d'un ligand à son RCPG permet l'activation de la PLC$\beta$ qui catalyse l'hydrolyse de phosphatidyl inositol diphosphate (PIP2) membranaire en diacylglycérol (DAG) qui reste membranaire et en inositol triphosphate (IP3) soluble. La libération d'IP3 dans le cytosol conduit à l'augmentation du calcium intracellulaire. En partenariat avec la phosphatidylsérine (PtdSer) membranaire, le DAG et le calcium formés entraînent le recrutement de la PKC$\beta$2 à la membrane par l'intermédiaire de ses domaines C1 et C2. Avantageusement pour l'invention, il a été démontré que le recrutement de la PKC$\beta$II peut être aussi induit directement par traitement des cellules par un ester de phorbol 12-myristoylé 13-acétylé (PMA), analogue structural du DAG, mais sans que cette translocation ne dépende de la liaison d'un ligand à son récepteur membranaire (RCPG).

Construction du plasmide codant pour la protéine Cycle3-GFP-PKC$\beta$II

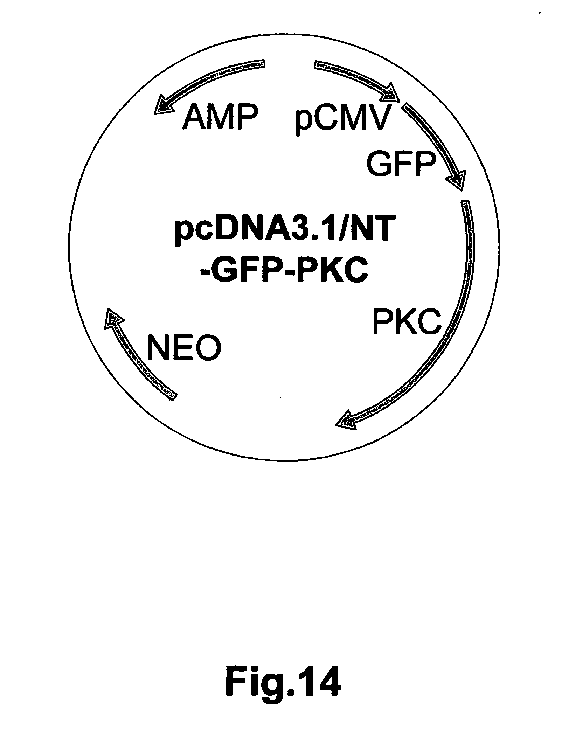

L'ADNc codant pour la protéine PKC$\beta$II (pAcMP1/PKCbetaII) est commercialisé par la société American Type Culture Collection (ATCC, Manassas, USA) sous la référence ATCC-80046. Le fragment PKCβII, contenant la séquence codante de la protéine PKCβII, est obtenu par digestion du vecteur pAcMP1/PKCbetaII par l'enzyme de restriction BamHI (New England Biolabs, Beverly, MA, USA) suivi d'un traitement par le fragment klenow de la polymérase I. Ce fragment PKCβII de 2041 paires de bases est cloné dans le vecteur d'expression pcDNA3.1 (Invitrogen Life technologies, Cergy Pontoise, France Réf V790-20) préalablement digéré par EcoRV puis par la phosphatase alcaline. Le vecteur intermédiaire pcDNA3.1/PKCβII ainsi obtenu est digéré par XhoI, traité par l'enzyme klenow et enfin digéré par NheI, libérant ainsi le fragment de 2131 paires de bases codant pour la protéine PKCβII. Ce fragment est ensuite cloné dans le vecteur pcDNA3.1/NT-GFP (Invitrogen Life technologies, Cergy Pontoise, France) préalablement digéré par les enzymes NheI (New England Biolabs, Beverly MA, U.S.A.) et EcoRV. On obtient le vecteur d'expression pcDNA3.1/NT-GFP-PKCII codant pour la protéine de fusion Cycle3-GFP-PKCβII dont la carte est représentée sur la figure 14.

_Mise en oeuvre des cellules Cos-7 et transfection des cellules avec l'ADN codant la protéine Cycle3-GFP-PKCβII en vue de l'observation des cellules vivantes en chambre de Rose_

On ensemence stérilement une boite de culture plastique de 24 $cm^2$ de surface (Nunclon®Delta, PolyLabo, Strasbourg, France) qui a été préalablement équipée d'une lamelle de verre ronde de 10 $cm^2$ de surface avec 800 000 cellules Cos-7 (ECACC n°87021302), cultivées au 10ème passage. Le milieu de culture (5 ml par boite de 24 $cm^2$ de surface) est du DMEM (Invitrogen LifeTechnologies, Cergy-Pontoise, France, Réf: 41966-029) complémenté avec 10% (v/v) de sérum de veau foetal (Sigma-Aldrich Chimie SARL, Saint-Quentin-Fallavier, France, Ref F7524), de la Penicilline à une concentration de 100 UI/ml et de la Streptomycine à une concentration de 100 μg/ml. 24 heures après l'ensemencement, les cellules sont tansfectées avec l'ADN plasmidique codant pour la Cycle3-GFP-PKCII en utilisant la LipofectAMINE®2000 (Invitrogen Life Technologies, Cergy-Pontoise, France, Réf 11668-019). Pour ce faire, on prépare 8 μg d'ADN plasmidique pcDNA3.1/NT-GFP-PKCII dans 500 μl de milieu DMEM. On homogénéise par vortex et on incube ce mélange pendant 5 minutes à 20°C. Par ailleurs, on prépare 20 μl de LipofectAMINE™2000 dans 500 μl de milieu DMEM. On homogénéise par vortex et on incube ce mélange pendant 5 minutes à 20°C. On transfère la solution de LipofectAMINE™2000 dans la solution d'ADN et on incube ce mélange pendant 5 minutes à 20°C. Pendant ce temps, on lave les cellules avec 3 ml de DMEM puis on leur ajoute 4 ml de DMEM et enfin, on ajoute le mélange de transfection (1 ml) sur les cellules. On homogénéise et on incube les cellules pendant 4 heures à 37°C sous 5% de $CO_2$. On élimine le milieu de transfection et on le remplace par 5 ml de milieu DMEM, complémenté avec 10% de sérum de veau foetal, de la penicilline et de la streptomycine. Les cellules sont replacées dans l'incubateur à 37°C sous 5% de $CO_2$ pendant 24 heures. Les cellules sont observées 24 heures après la fin de la transfection.

_Contrôle par imagerie de la translocation de la protéine Cycle3-GFP-PKCII lors du traitement des cellules vivantes avec un ester de phorbol (PMA)_

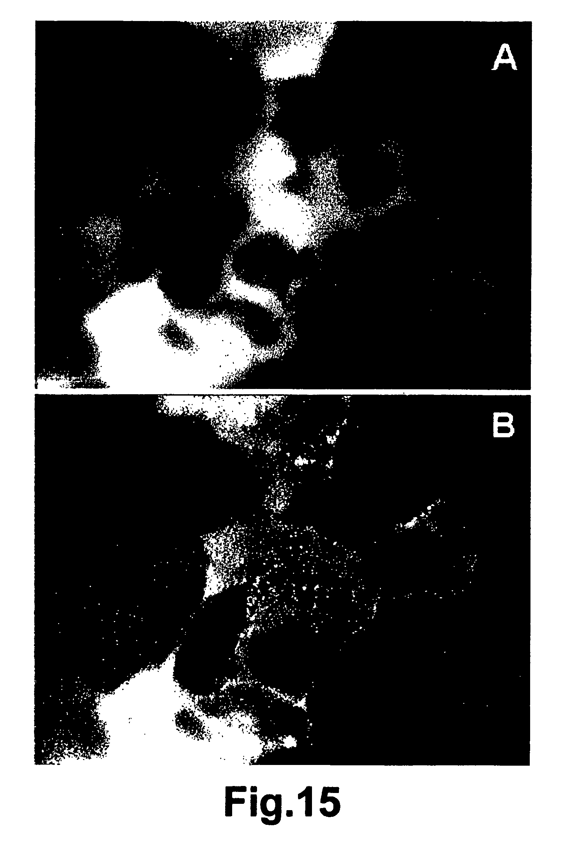

24 heures après la fin de la transfection, la lamelle supportant les cellules est placée dans une chambre de Rose en présence de milieu tampon KREBS (HEPES 25 mM, NaCl 118,4 mM, KCl 5 mM, $MgSO_4$ 1,18 mM, $CaCl_2$ 1,29 mM, Glucose 11,7 mM, pH 7,4). L'observation des cellules est réalisée au moyen d'un microscope à fluorescence droit (Zeiss Axioplan) équipé d'un objectif à immersion Gx40 et d'une caméra vidéo. La longueur d'onde d'excitation est de 400 nm (filtre d'excitation 405DF40, Omega Optical Inc, Brattleboro, Vermont, USA), la fluorescence est observée à 510 nm (filtre d'émission 510DF60, Omega Optical Inc, Brattleboro, Vermont, USA) et la lumière excitatrice est éliminée par réflexion en-deçà de 455 nm (filtre dichroïque 455DRLP, Omega Optical Inc, Brattleboro, Vermont, USA). On observe sur la figure 15 des cellules Cos-7 présentant une fluorescence répartie de façon homogène dans le cytoplasme des cellules à l'exception des noyaux qui apparaissent sombres. On induit le changement de localisation de la Cycle3-GFP-PKCβII en traitant les cellules avec une solution d'ester de phorbol 12-myristoylé 13-acétylé (4β,9α,12β,13α,20-pentahydroxytiglia-1,6-dien-3-one-12-tetradecanoate-13-acetate ou PMA) (Sigma-Aldrich Chimie SARL, Saint-Quentin-Fallavier, France, Ref P-1585). Pour ce faire, on aspire avec une seringue le milieu de culture présent dans la chambre de Rose et au temps t = 0 on injecte 1,5 ml de tampon KREBS contenant le PMA à une concentration finale de 16 μM. On replace la chambre de Rose sous l'objectif du microscope et on acquiert une image après une minute de traitement puis après dix minutes de traitement. Les clichés sont présentés sur la figure 15. On observe, entre une minute (Figure 15A) et dix minutes de traitement (Figure 15B) une forte diminution de la fluorescence de la Cycle3-GFP-PKCβII dans le cytoplasme des cellules transfectées et une apparition concomitante de la fluorescence au niveau des membranes plasmiques. L'épuisement du cytoplasme en Cycle3-GFP-PKCβII est en particulier caractérisé par la perte de contraste entre le cytoplasme et le noyau des cellules.

Analyse par FRET de la translocation vers la membrane plasmique de la protéine Cycle3-GFP-PKCβII lors du traitement des cellules avec un ester de phorbol (PMA)

• Marquage avec la sonde fluorescente DiI des membranes plasmiques de cellules Cos-7 en culture transfectées avec le plasmide codant pour la Cycle3-GFP-PKCβII.

On ensemence stérilement une boite de culture plastique de 24 cm2 de surface (Nunclon®Delta, PolyLabo, Strasbourg, France) qui a été préalablement garnie d'une lamelle de verre de 12 mm x 24 mm avec 200 000 cellules Cos-7 (ECACC n°87021302), cultivées au 2ième passages. 72 heures après, les cellules sont transfectées avec l'ADN codant pour la Cycle3-GFP-PKCII comme décrit plus haut. 24 heures après la fin de la transfection les cellules sont marquées avec la sonde membranaire DiI. Pour ce faire, on prépare une solution de dioctadécyl-1,1'-tétraméthyl-3,3,3', 3'-indocarbocyanine (DiI, Molecular Probes, Leiden, Pays-Bas, Réf: D-282) à une concentration de 10 $\mu$g/$\mu$l dans la diméthylformamide. On prépare par ailleurs le milieu de marquage comprenant 2,95 ml de milieu DMEM (Invitrogen LifeTechnologies, Cergy-Pontoise, France, Réf: 41966-029), 30 $\mu$l d'une solution d'albumine de sérum bovin dépourvue d'acides gras (Sigma-Aldrich Chimie SARL, Saint-Quentin-Fallavier, France, Ref A-8806) chauffé à 37°C et dans lequel on disperse, à 37°C, 15 $\mu$l de la solution de DiI décrite plus haut. La concentration en DiI dans le milieu de marquage est de 50 $\mu$g/ml. On aspire le milieu de culture de la boite et on lave les cellules avec 3 ml de DMEM. On incube 3 ml de milieu de marquage avec les cellules pendant 5 minutes à une température de 20°C. On élimine le milieu de marquage et on lave les cellules 3 fois avec 3 ml de tampon KREBS. La lamelle supportant les cellules est alors placée selon la diagonale d'une cuvette en quartz de 1 cm de côté adaptée à la fluorescence et dans laquelle on place 2,5 ml de tampon KREBS.

• Analyse par spectrofluorimétrie de la fluorescence émise par des cellules Cos-7 transfectées avec la protéine Cycle3-GFP-PKCβII et dont les membranes sont marquées avec la sonde DiC18.

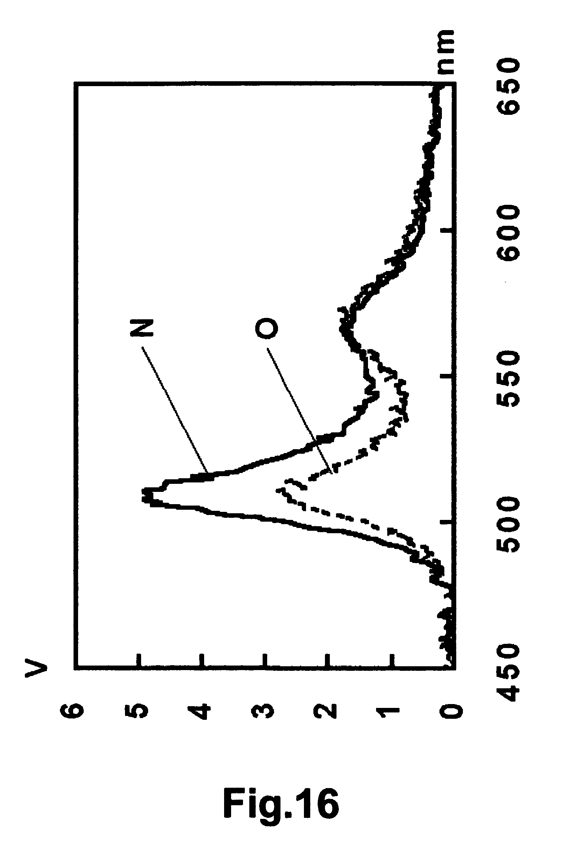

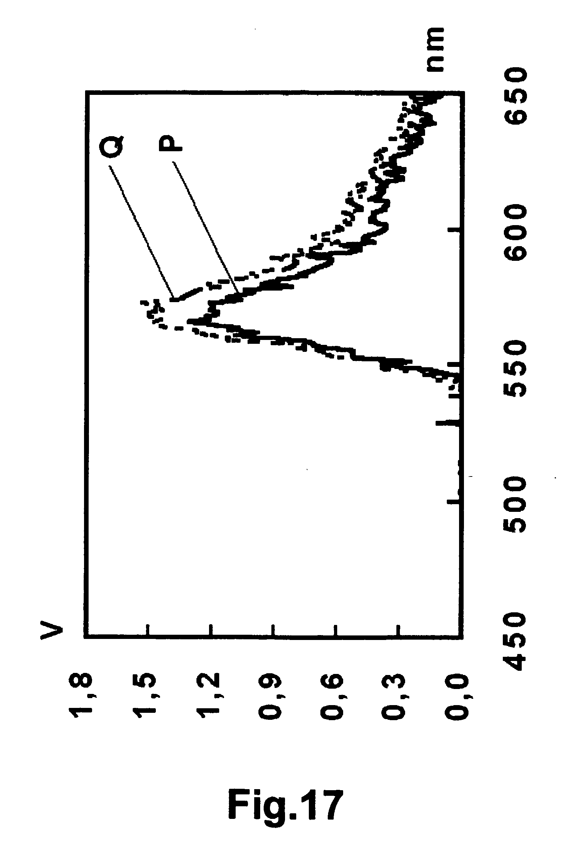

La cuvette est placée dans le spectrofluorimètre SPF-500C™ (SLM AMINCO, Inc ; BIORITECH ; France) et sa position est ajustée de façon à éviter la réflexion directe de la lumière excitatrice vers la fente d'entrée de l'analyseur du spectrofluorimètre. On acquiert ainsi entre 450 nm et 650 nm (bande passante de 5 nm) le spectre d'émission de fluorescence des cellules transfectées avec la protéine Cycle3-GFP-PKCβII et marquées avec la sonde DiI. La longueur d'onde d'excitation étant de 398 nm (bande passante de 7,5 nm). Le temps d'acquisition de ce spectre est de 55 secondes pour un pas d'incrémentation de 1,1 nm. Au temps t = 0, on ajoute dans la cuvette de fluorescence 500 $\mu$l d'une solution de PMA dans du tampon phosphate 10 mM, NaCl 150 mM, pH 7,4 à une concentration finale en PMA dans la cuvette de 16 $\mu$M. On enregistre ainsi un spectre toutes les minutes jusqu'à 20 minutes après l'induction. On enregistre ensuite un spectre d'émission de fluorescence d'une lamelle supportant des cellules non transfectées ni marquées avec le DiI que l'on soustrait à tous les spectres d'émission de fluorescence obtenus après l'induction au PMA. On représente sur la figure 16 les spectres d'émission de fluorescence (Intensité de fluorescence en fonction de la longueur d'onde, en nanomètres) corrigés observés au moment de l'induction (N) puis à 20 minutes après le moment de l'induction (O). Afin de visualiser l'augmentation simultanée de la fluorescence du DiI, on retranche aux spectres (N) et (O) la contribution propre de la Cycle3-GFP-PKCβII à la fluorescence totale. On trace ainsi les spectres d'émission de fluorescence propre du DiI analysés respectivement à 1 minute et 20 minutes après l'induction par le PMA. Le résultat est représenté sur la figure 17 qui montre une augmentation nette de la fluorescence de l'accepteur DiI entre l'état initial (P) et l'état final (Q). De plus, afin de qualifier le transfert de fluorescence par FRET, on analyse l'évolution de la fluorescence de la Cycle3-GFP-PKCβII après traitement des cellules avec une solution de PMA 16 $\mu$M en l'absence de l'accepteur membranaire de FRET DiI. Les spectres de fluorescence analysés respectivement aux temps 1 minute et 20 minutes après l'induction au PMA 16 $\mu$M restent identiques (résultats non montrés) alors qu'une analyse par microscopie de fluorescence montre une localisation membranaire de la Cycle3-GFP-PKCβII au temps 20 minutes après l'induction à 20°C.

• Quantification de l'effcacité du transfert de fluorescence entre la Cycle3-GFP-PKCβII et la sonde membranaire DiI au cours du recrutement de la Cycle3-GFP-PKCβII à la membrane plasmique induit par un traitement au PMA.

L'efficacité du transfert non radiatif de fluorescence entre la Cycle3-GFP-PKCβII et la sonde membranaire DiI est donnée comme le pourcentage de variation de la fluorescence comparée à la fluorescence initiale :

$$\text{FRET \%} = 100 \times ( I_0 - I_t ) / I_0$$

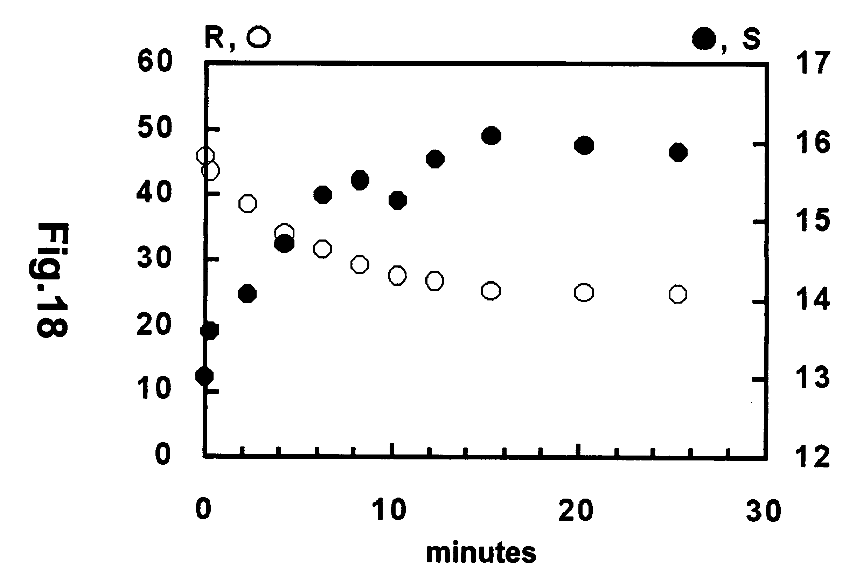

Où $I_0$ est la fluorescence initiale mesurée sur les cellules non induites et It est la fluorescence mesurée sur les cellules au temps t après traitement au PMA. En pratique, $I_0$ et $I_t$ sont calculées comme le rapport de la fluorescence de la Cycle3-GFP-PKCβII sur la fluorescence du DiI. La fluorescence de la Cycle3-GFP-PKCβII avant induction ou au temps t après induction est évaluée par intégration de son spectre de fluorescence entre 505 et 515 nm. La fluorescence du DiI avant induction ou au temps t après induction est évaluée par intégration de son spectre de fluorescence déconvolué entre 570 et 580 nm. On trace sur la Figure 18, les variations de la fluorescence de la Cycle3-GFP-PKCβII (R) et du DiI (S) au cours du temps (en minutes) à la suite du traitement des cellules par le PMA. Après 20 minutes de traitement, on note une perte de 45% du signal de la Cycle3-GFP-PKCβII ainsi que l'augmentation de 20% du signal du DiI. On remarque aussi que la perte de la fluorescence de la Cycle3-GFP-PKCβII est très fortement corrélée avec l'augmentation de la fluorescence du DiI avec un effet saturant à partir de 10 minutes de traitement au PMA.

La variation du FRET calculé à partir de ces valeurs et selon la formule donnée ci-dessus est représentée sur la Figure 18. L'efficacité du transfert de la fluorescence de la Cycle3-GFP-PKCβII vers le DiI varie de 0% au moment de l'induction de la translocation de la protéine encore cytosolique jusqu'à 55% après 17 minutes de traitement alors que la protéine est alors transloquée à la membrane plasmique. Ce résultat appuyé et renforcé par les observations faites par microscopie de fluorescence de la translocation de la protéine Cycle3-GFP-PKCβII vers la membrane plasmique de cellules eucaryotes en culture induite par le PMA démontre de façon claire le fort potentiel du procédé pour détecter la translocation d'une protéine fluorescente du cytosol dans l'environnement immédiat de la membrane plasmique au moyen de mesures quantitatives de fluorescence de la Cycle3-GFP-PKCβII et de l'accepteur DiI.

**Revendications**

1. Procédé de sélection d'agents, dits agents à tester, susceptibles d'être biologiquement actifs vis-à-vis d'un système cellulaire vivant en induisant un changement d'état biochimique d'un signal cellulaire à au moins deux états différents de la membrane cellulaire d'au moins une cellule vivante du système cellulaire vivant, dite cellule sensible, par utilisation d'un phénomène de couplage de luminescence entre au moins deux groupements photocomplémentaires, susceptible de générer et/ou de modifier au moins une émission lumineuse, dont au moins une propriété mesurable est spécifiquement dépendante de la distance séparant les groupements photocomplémentaires et est modifiée lorsque le signal cellulaire change d'état,
**caractérisé en ce que** :

- on prépare un système cellulaire vivant contenant au moins une cellule sensible,
- on introduit dans le système cellulaire ou on fait produire par le système cellulaire au moins un agent, dit agent intermédiaire marqué, choisi pour que, dans un premier état du signal cellulaire, cet agent intermédiaire marqué soit spécifiquement recruté à la membrane cellulaire d'au moins une cellule sensible, et pour que dans un deuxième état du signal cellulaire, cet agent intermédiaire marqué ne soit pas spécifiquement recruté par une membrane cellulaire de cellule sensible, ledit agent intermédiaire marqué étant une protéine naturelle marquée avec au moins un des groupements photocomplémentaires, dit premier luminophore, dans des conditions propres à préserver au moins sensiblement les propriétés du système cellulaire vivant vis-à-vis du signal cellulaire, cette protéine naturelle étant choisie parmi :

- une protéine de transduction intracellulaire spécifique de l'état d'au moins un récepteur membranaire susceptible d'être activé par au moins un des agents à tester, ledit signal cellulaire étant un signal de transduction intracellulaire dont le premier état correspond au recrutement spécifique de la protéine de transduction à la membrane cellulaire, la protéine de transduction et le premier luminophore étant choisis pour que la protéine de transduction marquée présente au moins les fonctionnalités et le comportement de la protéine de transduction dans l'expression du signal de transduction intracellulaire par la(les) cellule(s) sensible(s),
- une protéine intégrale de la membrane plasmique dont un fragment peut être libéré sous l'action de protéases,
- une protéine intracellulaire qui peut être transloquée vers la membrane d'organites intracellulaires,

- on introduit dans le système cellulaire vivant au moins une composition d'au moins un composé, dit agent membranaire de couplage, apte à s'associer à une membrane cellulaire de cellule sensible et marqué avec au moins un groupement photocomplémentaire, dit deuxième luminophore, distinct du premier luminophore, et de telle sorte que les propriétés du système cellulaire vivant vis-à-vis du signal cellulaire soient au moins sensiblement préservées et qu'au moins une propriété mesurable de l'émission lumineuse résultant du couplage de luminescence entre le premier et le deuxième luminophore soit sensiblement modifiée lorsque le signal cellulaire change d'état ; ledit agent membranaire de couplage comprenant un ligand lipophile choisi parmi les phospholipides, les acides gras, les glycérides, le cholestérol, les céramides, les amines grasses, les agents luminophores lipophiles ou les dérivés lipophiles de ces composés ;

- on met le système cellulaire vivant ainsi préparé en présence d'une quantité de l'un des agents à tester, dit agent en test, on mesure au moins ladite propriété mesurable, et on détermine l'activité biologique de cette quantité de l'agent en test en fonction du résultat de cette mesure.

**2.** Procédé selon la revendication 1, **caractérisé en ce que** les premier et deuxième luminophores sont choisis pour réaliser un couplage de luminescence par transfert d'énergie par résonance.

**3.** Procédé selon l'une des revendications 1 ou 2, **caractérisé en ce que** le premier et deuxième luminophores sont choisis pour réaliser un couplage de luminescence par transfert d'énergie de fluorescence par résonance (FRET).

**4.** Procédé selon l'une des revendications 1 à 3, **caractérisé en ce que** l'un au moins des premier et deuxième luminophores est choisi parmi :

- une molécule susceptible d'absorber la lumière émise par une protéine fluorescente,
- une protéine fluorescente naturelle ou une protéine fluorescente mutée, par addition, délétion ou substitution d'un ou plusieurs acides aminés d'une protéine fluorescente naturelle, cette protéine fluorescente mutée conservant la propriété de fluorescence,
- une protéine bioluminescente naturelle ou mutée,
- un fragment fluorescent issu d'une protéine fluorescente naturelle ou d'une protéine fluorescente mutée adapté pour présenter la propriété de fluorescence de la protéine fluorescente dont il est issu,
- la fluorescéine, la rhodamine, le Bodipy®, le Nile Red®, le Texas Red®, les chélates de terres rares, les indo et carbocyanines, le diphénylhexatriène, les dérivés aromatiques luminescents.

**5.** Procédé selon l'une des revendications 1 à 4, **caractérisé en ce que** l'un au moins des premier et deuxième luminophores est une protéine autofluorescente naturelle de cnidaire.

**6.** Procédé selon l'une des revendications 1 à 5, **caractérisé en ce qu'**on réalise le marquage de la protéine de transduction par voie génétique en faisant exprimer par au moins une cellule sensible du système cellulaire vivant, une protéine de fusion formant la protéine de transduction marquée.

**7.** Procédé selon l'une des revendications 1 à 6, **caractérisé en ce qu'**on choisit la protéine de transduction parmi les protéines contenant des domaines (notamment les domaines PH ou FYVE) interagissant avec des lipides membranaires ; les protéines s'associant à la membrane plasmique par ancrage lipidique, notamment de type N-myristoylation, S-palmitoylation, S-prénylation ; les protéines associées aux récepteurs couplés aux protéines G ; les protéines associées aux récepteurs à activité enzymatique tyrosine kinase intrinsèque ; les protéines STAT ; les protéines associées aux récepteurs à activité enzymatique sérine/thréonine kinase intrinsèque ; les protéines associées aux récepteurs à activité enzymatique tyrosine phosphatase intrinsèque ; les protéines JAK associées aux récepteurs reconnaissant des cytokines correspondantes ; les protéines associées aux récepteurs à domaine TIR ou DD ; les protéines associées aux récepteurs reconnaissant des antigènes.

**8.** Procédé selon l'une des revendications 1 à 7, **caractérisé en ce que** le système cellulaire vivant comprend au moins une cellule sensible eucaryote vivante.

**9.** Système cellulaire vivant à l'exception d'un embryon humain, d'un corps humain ou d'un élément non isolé d'un corps humain, comprenant au moins une cellule vivante, dite cellule sensible et comprenant :

- au moins un agent marqué, dit agent intermédiaire marqué ; ledit agent intermédiaire marqué étant une protéine naturelle marquée avec au moins un groupement photocomplémentaire, dit premier luminophore, et étant adapté pour être spécifiquement recruté à la membrane cellulaire dans un premier état du signal cellulaire et pour ne pas être spécifiquement recruté par une membrane cellulaire dans un deuxième état du signal cellulaire ; ladite protéine naturelle étant choisie parmi :

- une protéine de transduction intracellulaire spécifique de l'état d'au moins un récepteur membranaire susceptible d'être activé par au moins un des agents à tester, ledit signal cellulaire étant un signal de transduction intracellulaire dont le premier état correspond au recrutement spécifique de la protéine de transduction à la membrane cellulaire, la protéine de transduction et le premier luminophore étant choisis pour que la protéine de transduction marquée présente au moins les fonctionnalités et le comportement de la protéine de transduction dans l'expression du signal de transduction intracellulaire par la cellule sensible,

- une protéine intégrale de la membrane plasmique dont un fragment peut être libéré sous l'action de protéases,

- une protéine intracellulaire qui peut être transloquée vers la membrane d'organites intracellulaires,

- au moins un composé, dit agent membranaire de couplage, incorporé dans la membrane cellulaire et marqué avec au moins un groupement, dit deuxième luminophore, distinct dudit premier luminophore et photocomplémentaire dudit premier luminophore dans un phénomène de couplage de luminescence susceptible de générer et/ou de modifier au moins une émission lumineuse dont au moins une propriété mesurable est spécifiquement dépendante de la distance séparant les groupements photocomplémentaires ; l'agent membranaire de couplage étant associé à la membrane cellulaire en une quantité adaptée pour au moins sensiblement préserver les propriétés du système cellulaire vivant vis-à-vis d'un signal cellulaire à au moins deux états différents de la membrane cellulaire, et pour qu'au moins une propriété mesurable de l'émission lumineuse résultant du couplage de luminescence soit sensiblement modifiée lorsque le signal cellulaire change d'état ; l'agent membranaire de couplage ne correspondant pas à l'agent de la membrane cellulaire spécifiquement reconnu par un agent intermédiaire marqué lors de son recrutement à la membrane cellulaire et comprenant un ligand lipophile choisi parmi les phospholipides, les acides gras, les glycérides, le cholestérol, les céramides, les amines grasses, les agents luminophores lipophiles ou les dérivés lipophiles de ces composés.

**10.** Système cellulaire vivant selon la revendication 9, **caractérisé en ce qu'**au moins une cellule sensible, dont la membrane cellulaire incorpore au moins un agent membranaire de couplage, contient une séquence d'ADN comprenant un gène codant pour une protéine, dite protéine intermédiaire, choisie pour être spécifiquement recrutée à la membrane cellulaire de la(des) cellule(s) sensible(s) lorsque le signal cellulaire est dans un premier état, et pour ne pas être recrutée par la membrane cellulaire de la(des) cellule(s) sensible(s) lorsque le signal cellulaire est dans un deuxième état, fusionné avec au moins un gène codant pour un premier luminophore, la fusion de ces gènes étant telle que :

- lorsque le signal cellulaire est dans le premier état, une protéine de fusion, dite protéine intermédiaire marquée, formée de la protéine intermédiaire et du premier luminophore est exprimée par la(les) cellule(s) sensible(s) et recrutée à la membrane cellulaire, et fait office d'agent intermédiaire marqué,

- lorsque le signal cellulaire est dans le deuxième état, aucune protéine intermédiaire marquée n'est recrutée par une membrane cellulaire de cellule sensible.

**11.** Système selon l'une des revendications 9 ou 10, **caractérisé en ce que** les premier et deuxième luminophores sont choisis pour réaliser un couplage de luminescence par transfert d'énergie par résonance.

**12.** Système selon l'une des revendications 9 à 11, **caractérisé en ce que** le premier et deuxième luminophores sont choisis pour réaliser un couplage de luminescence par transfert d'énergie de fluorescence par résonance (FRET).

**13.** Système selon l'une des revendications 9 à 12, **caractérisé en ce que** l'un au moins des premier et deuxième luminophores est choisi parmi :

- une molécule susceptible d'absorber la lumière émise par une protéine fluorescente,

- une protéine fluorescente naturelle ou une protéine fluorescente mutée, par addition, délétion ou substitution d'un ou plusieurs acides aminés d'une protéine fluorescente naturelle, cette protéine fluorescente mutée conservant la propriété de fluorescence,

- une protéine bioluminescente naturelle ou mutée,

- un fragment fluorescent issu d'une protéine fluorescente naturelle ou d'une protéine fluorescente mutée adapté pour présenter la propriété de fluorescence de la protéine fluorescente dont il est issu,

- la fluorescéine, la rhodamine, le Bodipy®, le Nile Red®, le Texas Red®, les chélates de terres rares, les indo et carbocyanines, le diphénylhexatriène, les dérivés aromatiques luminescents.

**14.** Système selon l'une des revendications 9 à 13, **caractérisé en ce que** l'un au moins des premier et deuxième luminophores est une protéine autofluorescente naturelle de cnidaire.

**15.** Système selon l'une des revendications 9 à 14, **caractérisé en ce que** la protéine de transduction est marquée par voie génétique en faisant exprimer par au moins une cellule sensible du système cellulaire vivant, une protéine de fusion formant la protéine de transduction marquée.

16. Système selon l'une des revendications 9 à 15, **caractérisé en ce que** la protéine de transduction est choisie parmi les protéines contenant des domaines (notamment les domaines PH ou FYVE) interagissant avec des lipides membranaires ; les protéines s'associant à la membrane plasmique par ancrage lipidique, notamment de type N-myristoylation, S-palmitoylation, S-prénylation ; les protéines associées aux récepteurs couplés aux protéines G ; les protéines associées aux récepteurs à activité enzymatique tyrosine kinase intrinsèque ; les protéines STAT ; les protéines associées aux récepteurs à activité enzymatique sérine/thréonine kinase intrinsèque ; les protéines associées aux récepteurs à activité enzymatique tyrosine phosphatase intrinsèque ; les protéines JAK associées aux récepteurs reconnaissant des cytokines correspondantes ; les protéines associées aux récepteurs à domaine TIR ou DD ; les protéines associées aux récepteurs reconnaissant des antigènes.

17. Système selon l'une des revendications 9 à 16 **caractérisé en ce que** le système cellulaire vivant comprend au moins une cellule sensible eucaryote vivante.

**Claims**

1. Method for selecting agents, referred to as test agents, capable of being biologically active in respect of a living cell system by inducing a change in the biochemical state of a cell signal having at least two different states of the cell membrane of at least one living cell of the living cell system, referred to as the sensitive cell, by using a phenomenon of luminescence coupling between at least two photocomplementary groups, capable of generating and/or modifying at least one light emission, of which at least one measurable property is specifically dependent on the distance separating the photocomplementary groups and is modified when the cell signal changes state, **characterised in that**:

   - a living cell system containing at least one sensitive cell is prepared,
   - at least one agent, referred to as the labelled intermediate agent, is introduced into the cell system, or the cell system is made to produce at least one such agent, said labelled intermediate agent being chosen so that, in a first state of the cell signal, the labelled intermediate agent is specifically recruited to the cell membrane of at least one sensitive cell and so that, in a second state of the cell signal, the labelled intermediate agent is not specifically recruited by a cell membrane of a sensitive cell, said labelled intermediate agent being a natural protein labelled with at least one of the photocomplementary groups, referred to as the first luminophore, under conditions capable of at least substantially preserving the properties of the living cell system in respect of the cell signal, the natural protein being selected from:

     - a specific intracellular transduction protein of the state of at least one membrane receptor capable of being activated by at least one of the test agents, said cell signal being an intracellular transduction signal whose first state corresponds to the specific recruitment of the transduction protein to the cell membrane, the transduction protein and the first luminophore being chosen so that the labelled transduction protein exhibits at least the functionalities and the behaviour of the transduction protein in the expression of the intracellular transduction signal by the sensitive cell(s),
     - an integral plasmic membrane protein, a fragment of which can be released under the action of proteases,
     - an intracellular protein which can be translocated to the membrane of intracellular organites,

   - there is introduced into the living cell system at least one composition of at least one compound, referred to as the membrane coupling agent, capable of associating with a cell membrane of a sensitive cell and labelled with at least one photocomplementary group, referred to as the second luminophore, distinct from the first luminophore, and in such a manner that the properties of the living cell system in respect of the cell signal are at least substantially preserved and that at least one measurable property of the light emission resulting from the luminescence coupling between the first and second luminophores is substantially modified when the cell signal changes state; said membrane coupling agent comprising a lipophilic ligand selected from the phospholipids, fatty acids, glycerides, cholesterol, ceramides, fatty amines, lipophilic luminophoric agents, and lipophilic derivatives of those compounds;
   - the living cell system so prepared is brought into the presence of a quantity of one of the test agents, referred to as the agent under test, at least said measurable property is measured, and the biological activity of that quantity of the agent under test is determined as a function of the result of that measurement.

**2.** Method according to claim 1, **characterised in that** the first and second luminophores are chosen to effect a luminescence coupling by resonance energy transfer.

**3.** Method according to either claim 1 or claim 2, **characterised in that** the first and second luminophores are chosen to effect a luminescence coupling by fluorescence resonance energy transfer (FRET).

**4.** Method according to any one of claims 1 to 3, **characterised in that** at least one of the first and second luminophores is selected from:

- a molecule capable of absorbing the light emitted by a fluorescent protein,
- a natural fluorescent protein, or a fluorescent protein mutated by addition, deletion or substitution of one or more amino acids of a natural fluorescent protein, the mutated fluorescent protein retaining the property of fluorescence,
- a natural or mutated bioluminescent protein,
- a fluorescent fragment which has been obtained from a natural fluorescent protein or from a mutated fluorescent protein and which is suitable for exhibiting the property of fluorescence of the fluorescent protein from which it has been obtained,
- fluorescein, rhodamine, Bodipy®, Nile Red®, Texas Red®, rare earth chelates, indo- and carbocyanines, diphenylhexatriene, luminescent aromatic derivatives.

**5.** Method according to any one of claims 1 to 4, **characterised in that** at least one of the first and second luminophores is a natural autofluorescent cnidarian protein.

**6.** Method according to any one of claims 1 to 5, **characterised in that** labelling of the transduction protein is effected genetically by making at least one sensitive cell of the living cell system express a fusion protein forming the labelled transduction protein.

**7.** Method according to any one of claims 1 to 6, **characterised in that** the transduction protein is selected from proteins containing domains (especially the PH or FYVE domains) that interact with membrane lipids; proteins that associate with the plasmic membrane by lipid anchoring, especially of the N-myristoylation, S-palmitoylation, S-prenylation type; proteins associated with receptors coupled to the G proteins; proteins associated with receptors having intrinsic tyrosine kinase enzymatic activity; STAT proteins; proteins associated with receptors having intrinsic serine/threonine kinase enzymatic activity; proteins associated with receptors having intrinsic tyrosine phosphatase enzymatic activity; JAK proteins associated with receptors that recognise corresponding cytokines; proteins associated with receptors having a TIR or DD domain; proteins associated with receptors that recognise antigens.

**8.** Method according to any one of claims 1 to 7, **characterised in that** the living cell system comprises at least one living eukaryotic sensitive cell.

**9.** Living cell system, with the exception of a human embryo, a human body or an element not isolated from a human body, comprising at least one living cell, referred to as the sensitive cell and comprising:

- at least one labelled agent, referred to as the labelled intermediate agent; said labelled intermediate agent being a natural protein labelled with at least one photocomplementary group, referred to as the first luminophore, and being adapted to be specifically recruited to the cell membrane in a first state of the cell signal and to be not specifically recruited by a cell membrane in a second state of the cell signal; said natural protein being selected from:

- a specific intracellular transduction protein of the state of at least one membrane receptor capable of being activated by at least one of the test agents, said cell signal being an intracellular transduction signal whose first state corresponds to the specific recruitment of the transduction protein to the cell membrane, the transduction protein and the first luminophore being chosen so that the labelled transduction protein exhibits at least the functionalities and the behaviour of the transduction protein in the expression of the intracellular transduction signal by the sensitive cell,
- an integral plasmic membrane protein, a fragment of which can be released under the action of proteases,
- an intracellular protein which can be translocated to the membrane of intracellular organites,

- at least one compound, referred to as the membrane coupling agent, incorporated into the cell membrane and labelled with at least one group, referred to as the second luminophore, which is distinct from said first luminophore and is photocomplementary to said first luminophore in a phenomenon of luminescence coupling capable of generating and/or modifying at least one light emission of which at least one measurable property is specifically dependent on the distance separating the photocomplementary groups; the membrane coupling agent being associated with the cell membrane in a quantity suitable for at least substantially preserving the properties of the living cell system in respect of a cell signal having at least two different states of the cell membrane, and so that at least one measurable property of the light emission resulting from the luminescence coupling is substantially modified when the cell signal changes state; the membrane coupling agent not corresponding to the agent of the cell membrane specifically recognised by a labelled intermediate agent on its recruitment to the cell membrane and comprising a lipophilic ligand selected from the phospholipids, fatty acids, glycerides, cholesterol, ceramides, fatty amines, lipophilic luminophoric agents, and lipophilic derivatives of those compounds.

10. Living cell system according to claim 9, **characterised in that** at least one sensitive cell, the cell membrane of which incorporates at least one membrane coupling agent, contains a DNA sequence comprising a gene coding for a protein, referred to as the intermediate protein, selected to be specifically recruited to the cell membrane of the sensitive cell(s) when the cell signal is in a first state, and not to be recruited by the cell membrane of the sensitive cell(s) when the cell signal is in a second state, fused to at least one gene coding for a first luminophore, the fusion of those genes being such that:

- when the cell signal is in the first state, a fusion protein, referred to as the labelled intermediate protein, formed by the intermediate protein and the first luminophore, is expressed by the sensitive cell(s) and recruited to the cell membrane, and acts as the labelled intermediate agent,
- when the cell signal is in the second state, no labelled intermediate protein is recruited by a cell membrane of a sensitive cell.

11. System according to either claim 9 or claim 10, **characterised in that** the first and second luminophores are chosen to effect a luminescence coupling by resonance energy transfer.

12. System according to any one of claims 9 to 11, **characterised in that** the first and second luminophores are chosen to effect a luminescence coupling by fluorescence resonance energy transfer (FRET).

13. System according to any one of claims 9 to 12, **characterised in that** at least one of the first and second luminophores is selected from:

- a molecule capable of absorbing the light emitted by a fluorescent protein,
- a natural fluorescent protein, or a fluorescent protein mutated by addition, deletion or substitution of one or more amino acids of a natural fluorescent protein, the mutated fluorescent protein retaining the property of fluorescence,
- a natural or mutated bioluminescent protein,
- a fluorescent fragment which has been obtained from a natural fluorescent protein or from a mutated fluorescent protein and which is suitable for exhibiting the property of fluorescence of the fluorescent protein from which it has been obtained,
- fluorescein, rhodamine, Bodipy®, Nile Red®, Texas Red®, rare earth chelates, indo- and carbocyanines, diphenylhexatriene, luminescent aromatic derivatives.

14. System according to any one of claims 9 to 13, **characterised in that** at least one of the first and second luminophores is a natural autofluorescent cnidarian protein.

15. System according to any one of claims 9 to 14, **characterised in that** the transduction protein is labelled genetically by making at least one sensitive cell of the living cell system express a fusion protein forming the labelled transduction protein.

16. System according to any one of claims 9 to 15, **characterised in that** the transduction protein is selected from proteins containing domains (especially the PH or FYVE domains) that interact with membrane lipids; proteins that associate with the plasmic membrane by lipid anchoring, especially of the N-myristoylation, S-palmitoylation, S-prenylation type; proteins associated with receptors coupled to the G proteins; proteins associated with receptors having intrinsic tyrosine kinase enzymatic activity; STAT proteins; proteins associated with receptors having intrinsic serine/threonine kinase enzymatic activity; proteins associated with receptors having intrinsic tyrosine phosphatase enzymatic activity; JAK proteins associated with receptors that recognise corresponding cytokines; proteins associated with receptors having a TIR or DD domain; proteins associated with receptors that recognise antigens.

17. System according to any one of claims 9 to 16, **characterised in that** the living cell system comprises at least one living eukaryotic sensitive cell.

**Patentansprüche**

1. Verfahren zur Auswahl von Wirkstoffen, bezeichnet als zu testende Wirkstoffe, die geeignet sind, biologisch gegenüber einem lebenden Zellensystem durch Induzieren einer biochemischen Zustandsänderung eines Zellsignals von wenigstens zwei unterschiedlichen Zellmembranzuständen wenigstens einer lebenden Zelle des lebenden Zellensystems, bezeichnet als sensible Zelle, durch das Heranziehen eines Lumineszenzkopplungsphänomens zwischen wenigstens zwei fotokomplementären Gruppierungen aktiv zu sein, die geeignet sind, wenigstens eine Lichtemission zu generieren und / oder zu modifizieren, von der wenigstens eine messbare Eigenschaft spezifisch von der die fotokomplementären Gruppierungen trennenden Entfernung abhängt und modifiziert ist, wenn das Zellsignal den Zustand ändert,

   **dadurch gekennzeichnet, dass**:

   - ein wenigstens eine sensible Zelle enthaltendes lebendes Zellensystem hergestellt wird,
   - in das Zellensystem, in dem durch das Zellensystem wenigstens ein Wirkstoff, bezeichnet als **gekennzeichnet**er intermediärer Wirkstoff, eingeführt oder produziert wird, der ausgewählt ist, damit dieser **gekennzeichnet**e intermediäre Wirkstoff in einem ersten Zustand des Zellensystems spezifisch an der Zellmembran der wenigstens einen sensiblen Zelle rekrutiert wird und damit dieser **gekennzeichnet**e Zwischenwirkstoff in einem zweiten Zustand des Zellensystems nicht spezifisch durch eine Zellmembran einer sensiblen Zelle rekrutiert wird, wobei der genannte **gekennzeichnet**e intermediäre Wirkstoff ein **gekennzeichnet**es natürliches Protein mit wenigstens einer der als erster Luminophor bezeichneten fotokomplementären Gruppierungen unter Bedingungen ist, die geeignet sind, um wenigstens deutlich die Eigenschaften des lebenden Zellensystems gegenüber dem Zellsignal zu bewahren, wobei dieses natürliche Protein ausgewählt ist aus:

      - einem für den Zustand wenigstens eines zur Aktivierung wenigstens durch einen der zu testenden Wirkstoffe geeigneten Membranrezeptoren spezifischen intrazellulären Transduktionsprotein, wobei das genannte Zellsignal ein intrazelluläres Transduktionssignal ist, dessen erster Zustand der spezifischen Rekrutierung des Transduktionsproteins an der Zellmembran entspricht, wobei das Transduktionsprotein und der erste Luminophor ausgewählt sind, damit das **gekennzeichnet**e Transduktionsprotein wenigstens die Funktionalitäten und das Verhalten des Transduktionsproteins in der Expression des intrazellulären Transduktionsproteins durch die sensible(n) Zelle(n) aufweist,
      - ein integrales Protein der plasmischen Membran, von der ein Fragment unter der Proteasewirkung freigesetzt werden kann,
      - ein intrazelluläres Protein, das zur Membran intrazellulärer Organellen verschoben werden kann,
      - in das lebende Zellensystem wenigstens eine Zusammensetzung aus wenigstens einer Verbindung, bezeichnet als Kopplungsmembranwirkstoff, eingeführt wird, die geeignet ist, sich mit einer Zellmembran einer sensiblen Zelle zu verbinden und mit wenigstens einer fotokomplementären Gruppierung, bezeichnet als zweiter Luminophor, **gekennzeichnet** ist, der vom ersten Luminophor unterschiedlich ist, und derart, dass die Eigenschaften des lebenden Zellensystems gegenüber dem Zellsignal wenigstens deutlich gewahrt bleiben und dass wenigstens eine messbare Eigenschaft der aus der Lumineszenzkopplung zwischen dem ersten und dem zweiten Luminophor resultierenden Lichtemission deutlich modifiziert wird, wenn das Zellsignal den Zustand ändert; wobei der genannte Kopplungsmembranwirkstoff einen aus den Phospholipiden, Fettsäuren, Glyceriden, dem Cholesterin, den Ceramiden, den fetten Aminen, den lipophilen Luminophor-Wirkstoffen oder den lipophilen Derivaten dieser Verbindungen ausgewählten lipophilen Liganden umfasst;
      - das lebende Zellensystem wird somit bei Vorhandensein einer Menge eines der zu testenden Wirkstoffe, bezeichnet als Wirkstoff im Test, hergestellt, es wird wenigstens die messbare Eigenschaft gemessen, und es wird die biologische Aktivität dieser Menge des Wirkstoffs im Test in Abhängigkeit des Ergebnisses dieser Messung bestimmt.

2. Verfahren gemäß Anspruch 1, **dadurch gekennzeichnet, dass** die ersten und zweiten Luminophore ausgewählt sind, um eine Lumineszenzkopplung per Energietransfer per Resonanz zu realisieren.

3. Verfahren gemäß einem der Ansprüche 1 oder 2, **dadurch gekennzeichnet, dass** der erste und der zweite Luminophor ausgewählt sind, um eine Lumineszenzkopplung per Fluoreszenzenergietransfer per Resonanz (FRET) zu realisieren.

4. Verfahren gemäß einem der Ansprüche 1 bis 3, **dadurch gekennzeichnet, dass** wenigstens einer der ersten und zweiten Luminophore ausgewählt ist aus:

   - einem Molekül, das geeignet ist, das von einem fluoreszierenden Protein ausgegebene Licht zu absorbieren,
   - einem natürlichen fluoreszierenden Protein oder einem mutierten fluoreszierenden Protein durch Hinzufügen, Deletion oder Substitution einer oder mehrerer Aminosäure(n) eines natürlichen fluoreszierenden Proteins, wobei dieses mutierte fluoreszierende Protein die Fluoreszenzeigenschaft
   - eines natürlichen oder mutierten Biolumineszenzproteins,
   - eines Fluoreszenzfragments beibehält, das aus einem natürlichen fluoreszierenden Protein oder einem mutierten fluoreszierenden Protein stammt, das angepasst ist, um die Fluoreszenzeigenschaft des fluoreszierenden Proteins aufzuweisen, von dem es stammt,
   - dem Fluorescein, dem Rhodamin, dem Bodipy®, dem Nile Red®, dem Texas Red®, den Chelaten seltener Erden, den Indo- und Carbocyaninen, dem Diphenylhexatrien, den aromatischen Lumineszenzaromaten.

5. Verfahren gemäß einem der Ansprüche 1 bis 4, **dadurch gekennzeichnet, dass** wenigstens einer der ersten und zweiten Luminophore ein natürliches autofluoreszierendes Protein aus Nesseltieren ist.

6. Verfahren gemäß einem der Ansprüche 1 bis 5, **dadurch gekennzeichnet, dass** die Kennzeichnung des Transduktionsproteins auf genetischem Wege unter Ausdrücken durch wenigstens eine sensible Zelle des lebenden Zellensystems erfolgt, wobei ein Fusionsprotein das **gekennzeichnet**e Transduktionsprotein bildet.

7. Verfahren gemäß einem der Ansprüche 1 bis 6, **dadurch gekennzeichnet, dass** das Transduktonsprotein aus den Proteinen ausgewählt wird, das Bereiche (insbesondere die Bereiche PH oder FYVE) enthält, die mit Membranlipiden zusammenwirken; die sich per Lipidverankerung an die plasmische Membran verbindende Proteine, insbesondere vom Typ N-Myristoylation, S-Palmitoylation, S-Prenylation; die den an die Proteine G gekoppelten Rezeptoren zugeordneten Proteine; die den Rezeptoren mit intrinsischer, enzymatischer Kinase-Tyrosin-Aktivität zugeordneten Proteinen; die Proteine STAT; die den Rezeptoren mit intrinsischer enzymatischer Kinase-Threonin-/ -Serin-Aktivität zugeordnete Proteine; die den Rezeptoren mit intrinsischer enzymatischer Phosphatase-Tyrosin-Aktivität zugeordneten Proteine; die den Rezeptoren zugeordneten Proteine JAK, die entsprechende Zytokine erkennen; die den Rezeptoren mit TIR- oder DD-Bereich zugeordneten Proteine; die den Antigene erkennenden Rezeptoren zugeordneten Proteine.

8. Verfahren gemäß einem der Ansprüche 1 bis 7, **dadurch gekennzeichnet, dass** das lebende Zellensystem wenigstens eine sensible lebende eukaryotische Zelle umfasst.

9. Lebendes Zellensystem mit Ausnahme eines menschlichen Embryos, eines menschlichen Körpers oder eines nicht von einem menschlichen Körper isolierten Elements, das wenigstens eine lebende Zelle umfasst, bezeichnet als sensible Zelle, die Folgendes umfasst:

   - wenigstens einen **gekennzeichnet** Wirkstoff, bezeichnet als **gekennzeichnet**er intermediärer Wirkstoff; wobei der genannte **gekennzeichnet**e intermediäre Wirkstoff ein **gekennzeichnet**es natürliches Protein mit wenigstens einer fotokomplementären Gruppierung, bezeichnet als Luminophor, ist und angepasst ist, um spezifisch an der Zellmembran in einem ersten Zustand des Zellsignals rekrutiert zu werden und um nicht spezifisch durch eine Zellmembran in einem zweiten Zustand des Zellsignals rekrutiert zu werden; wobei das genannte natürliche Protein ausgewählt ist aus:
   - einem spezifischen intrazellulären Transduktionsprotein des Zustandes wenigstens eines Membranrezeptoren, der geeignet ist, durch wenigstens einen der zu testenden Wirkstoffe aktiviert zu werden, wobei das genannte Zellsignal ein intrazelluläres Transduktionssignal ist, dessen erster Zustand der spezifischen Rekrutierung des Transduktionsproteins an der Zellmembran entspricht, wobei das Transduktionsprotein und der erste Luminophor ausgewählt sind, damit das **gekennzeichnet**e Transduktionsprotein wenigstens die Funktionalitäten und das Verhalten des Transduktionsproteins in der Expression des intrazellulären Transduktionssignals durch die sensible Zelle aufweist,

- einem integralen Protein der plasmischen Membran, von dem ein Fragment unter der Proteasewirkung freigesetzt werden kann,
- ein intrazelluläres Protein, das zur Membran intrazellulärer Organellen verschoben werden kann,
- wenigstens eine Verbindung, bezeichnet als Kopplungsmembranwirkstoff, der in die Zellmembran integriert und mit wenigstens einer Gruppierung, bezeichnet als zweiter Luminophor, **gekennzeichnet** ist, der vom ersten Luminophor unterschiedlich ist und zu dem genannten ersten Luminophor in einem Lumineszenzkopplungsphänomen fotokomplementär ist, das geeignet ist, wenigstens eine Lichtausgabe zu generieren und / oder zu modifizieren, von der wenigstens eine messbare Eigenschaft spezifisch von der die fotokomplementären Gruppierungen trennenden Entfernung abhängt; wobei der Kopplungsmembranwirkstoff der Zellmembran in einer angepassten Menge zugeordnet ist, um wenigstens deutlich die Eigenschaften des lebenden Zellensystems gegenüber einem Zellsignal mit wenigstens zwei unterschiedlichen Zuständen der Zellmembran zu bewahren und damit wenigstens eine messbare Eigenschaft der aus der Lichtkopplung resultierenden Lichtausgabe deutlich modifiziert wird, wenn das Zellsignal den Zustand ändert; wobei der Kopplungsmembranwirkstoff nicht dem Wirkstoff der Zellmembran entspricht, der spezifisch durch einen **gekennzeichnet**en intermediären Wirkstoff bei seiner Rekrutierung an der Zellmembran erkannt wird und einen lipophilen Liganden umfasst, der aus den Phospholipiden, den Fettsäuren, den Glyceriden, dem Cholesterol, den Ceramiden, den fetten Aminen, den lipophilen Luminophor-Wirkstoffen oder den lipophilen Derivaten dieser Verbindungen ausgewählt sind.

**10.** Lebendes Zellensystem gemäß Anspruch 9, **dadurch gekennzeichnet, dass** wenigstens eine sensible Zelle, deren Zellmembran wenigstens einen Kopplungsmembranwirkstoff umfasst, eine DNA-Sequenz mit einem Gen enthält, das für ein Protein, bezeichnet als intermediäres Protein, kodiert, das ausgewählt ist, um spezifisch an der Zellmembran der sensiblen Zelle(n) rekrutiert zu werden, wenn das Zellsignal sich in einem ersten Zustand befindet, und um nicht von der Zellmembran der sensiblen Zelle(n) rekrutiert zu werden, wenn das Zellsignal sich in einem zweiten Zustand befindet, wobei er mit wenigstens einem für einen ersten Luminophor kodierenden Gen fusioniert ist, wobei die Fusion dieser Gene derart ist, dass:

- wenn das Zellsignal sich im ersten Zustand befindet, ein Fusionsprotein, bezeichnet als **gekennzeichnet**es intermediäres Protein, das vom intermediären Protein und dem ersten Luminophor gebildet wird, durch die sensible(n) Zelle(n) ausgedrückt und an der Zellmembran rekrutiert wird und als **gekennzeichnet**er Wirkstoff wirkt,
- wenn das Zellsignal sich im zweiten Zustand befindet, kein **gekennzeichnet**es, intermediäres Protein durch eine Zellmembran einer sensiblen Zelle rekrutiert wird.

**11.** System gemäß einem der Ansprüche 9 oder 10, **dadurch gekennzeichnet, dass** die ersten und zweiten Luminophore ausgewählt sind, um eine Lumineszenzkopplung per Energietransfer per Resonanz zu realisieren.

**12.** System gemäß einem der Ansprüche 9 oder 11, **dadurch gekennzeichnet, dass** der erste und der zweite Luminophor ausgewählt sind, um eine Lumineszenzkopplung per Fluoreszenz-Energietransfer per Resonanz (FRET) zu realisieren.

**13.** System gemäß einem der Ansprüche 9 bis 12, **dadurch gekennzeichnet, dass** wenigstens einer der ersten und zweiten Luminophoren ausgewählt ist aus:

- einem Molekül, das geeignet ist, das von einem fluoreszierenden Protein ausgegebene Licht zu absorbieren,
- einem natürlichen fluoreszierenden Protein oder einem mutierten fluoreszierenden Protein durch Hinzufügen, Deletion oder Substitution einer oder mehrerer Aminosäuren eines natürlichen fluoreszierenden Proteins, wobei dieses mutierte fluoreszierende Protein die Fluoreszenzeigenschaft beibehält,
- einem natürlichen oder mutierten Biolumineszenzprotein,
- einem fluoreszierenden Fragment, das aus einem natürlichen fluoreszierenden Protein oder einem mutierten fluoreszierenden Protein stammt, das geeignet ist, um die Fluoreszenzeigenschaft des fluoreszierenden Proteins aufzuweisen, aus dem es stammt,
- Fluorescein, Rhodamin, Bodipy®, Nile Red®, Texas Red@, Chelate seltener Erde, Indo- und Carbocyanine, Diphenylhexatrien, den aromatischen Lumineszenzderivaten.

**14.** System gemäß einem der Ansprüche 9 bis 13, **dadurch gekennzeichnet, dass** wenigstens einer der ersten und zweiten Luminophore ein natürliches autofluoreszierendes Protein aus Nesseltiere ist.

**15.** System gemäß einem der Ansprüche 9 bis 14, **dadurch gekennzeichnet, dass** das Transduktionsprotein auf genetischem Wege **gekennzeichnet** ist, indem durch wenigstens eine sensible Zelle des lebenden Zellensystems ein das **gekennzeichnet**e Transduktionsprotein bildendes Fusionsprotein ausgedrückt wird.

16. System gemäß einem der Ansprüche 9 bis 15, **dadurch gekennzeichnet, dass** das Transduktionsprotein aus den Proteinen ausgewählt wird, das mit Membranlipiden zusammenwirkende Bereiche (insbesondere die Bereiche PH oder FYVE) enthält; wobei die Proteine sich mit der plasmischen Membran per Lipidverankerung verbinden, insbesondere vom Typ N-Myristoylation, S-Palmitoylation, S-Prenylation; die den mit den Proteinen G gekoppelten Rezeptoren zugeordneten Proteine; die den Rezeptoren mit intrinsischer, enzymatischer Kinase-Tyrosin-Aktivität zugeordneten Proteine; die Proteine SAT; die den Rezeptoren mit intrinsischer enzymatischer Serin- / Threoninkinase-Aktivität zugeordneten Proteine; die den Rezeptoren mit intrinsischer, enzymatischer, Tyrosin-Phosphatase-Aktivität zugeordneten Proteine; die den Rezeptoren zugeordnete Proteine JAK, die entsprechende Zytokine erkennen; die den Rezeptoren mit TIR- oder DD-Bereich zugeordneten Proteine; die den Rezeptoren zugeordneten Proteine, die Antigene erkennen.

17. System gemäß einem der Ansprüche 9 bis 16, **dadurch gekennzeichnet, dass** das lebende Zellensystem wenigstens eine lebende eukaryotische sensible Zelle umfasst.

## Fig.1

Fig.2

Fig.3

Fig.4

Fig.5

**Fig.6**

Fig.7

Fig.8

Fig.9

**Fig.10**

Fig.11

Fig.12

Fig.13

**Fig.14**

**Fig.15**

Fig.16

Fig.17

Fig.18

Fig.19

## RÉFÉRENCES CITÉES DANS LA DESCRIPTION

**Documents brevets cités dans la description**

- WO 9507463 A
- WO 9623898 A
- WO 9845704 A
- WO 9855873 A
- WO 9912033 A
- WO 0043780 A

**Littérature non-brevet citée dans la description**

- **LAKOVICZ JR.** Principles of Fluorescence Spectroscopy. Kluwer Academic/Plenum Publishers, 1999, 368-391
- **ANGERS S ; SALAHPOUR A ; JOLY E ; HILAIRET S ; CHELSKY D ; DENNIS M ; BOUVIER M.** *Proceeding of the National Academy of Science USA,* 2000, vol. 97 (7), 3684-3689
- **LAKEY et al.** *J. Mol. Biol.,* 1991, vol. 1218, 639-653
- **V.TH. FÔRSTER.** *Ann. Phys.,* 1948, vol. 2, 54-75
- **J.R. LAKOVICZ.** Principles of Fluorescence Spectroscopy. Kluwer Academic/Plenum Publishers, 1999, vol. 13, 368-391
- **SHIMOMURA et al.** *FEBS Letters,* 2000, vol. 481, 19-25
- Rare Earth Cryptates and TRACE Technology as Tools for Probing Molecular Interactions in Biology. **ALPHA-BAZIN A ; BAZIN H ; PRÉAUDAT M ; TRINQUET E ; MATHIS G.** In New Trends in Fluorescence Spectroscopy - Applications to Chemical and Life Sciences. Springer-Verlag, 2001, vol. 21, 439-455
- **ROSE G.** *Tex. Rep. Biol. Med.,* 1954, vol. 12, 1074-1083